# EUROPEAN PATENT APPLICATION

(11) **EP 4 215 601 A1**
(43) Date of publication of application: **26.07.2023**
(21) Application number: 22152702.1
(22) Date of filing: 21.01.2022
(51) Int. Cl.: C12N 1/32, C12N 9/12

(54) **GENETICALLY MODIFIED BACTERIA RESISTANT TO MASS CELL LYSIS**

(71) Applicant: Acies Bio d.o.o., 1000 Ljubljana (SI)
(72) Inventor: Virant, David, Dob (SI); Kruis, Aleksander Johannes, Bled (SI); Nagode, Toni, Ljubljana (SI); Kosec, Gregor, Ljubljana (SI)
(74) Representative: Zacco GmbH

(57) **Abstract**

The present invention generally relates to the biotechnology engineering, and specifically to genetically modified bacteria of the genus *Methylobacillus* which have improved properties making them particularly useful in large scale methanol fermentations. More specifically, the present invention provides a bacterium of the genus *Methylobacillus* which has been modified to have a decreased production of exopolysaccharides (EPS) compared to an otherwise identical bacterium that does not carry said modification. The present invention further provides a method for producing a biochemical compound using a genetically modified bacterium of the present invention.

## Description

### Technical field of the invention

The present invention generally relates to the biotechnology engineering, and specifically to genetically modified bacteria which show resistance to mass cell lysis, making them particularly useful in large scale fermentations. More specifically, the present invention provides a bacterium modified to have a decreased expression and/or activity of an endogenous polypeptide having polyphosphate kinase activity compared to an otherwise identical bacterium that does not carry said modification. The present invention further provides a method for producing a biochemical compound using a genetically modified bacterium of the present invention.

### Background of the invention

Biotechnological chemical production through microbial fermentation is a more sustainable and environmentally friendly alternative to oil and other fossil fuel-based production. Several sustainable fermentation substrates have been used commercially for chemical production, mostly C6 and C5 carbohydrates, such as glucose and xylose. Commonly used organisms in such fermentations are, among others, *Escherichia coli, Bacillus subtilis,* and *Corynebacterium glutamicum.*

While glucose-based are sustainable, transitioning all chemical production to glucose-based fermentation while would require enormous of arable land just to produce enough feedstock. One way to circumvent this and still achieve sustainable chemical production is to replace glucose with a less land-intensive feedstock. Methanol is a possible candidate for this. It can be produced from carbon dioxide and hydrogen, bypassing the need for land. It can be easily stored, transported and is more energy-dense than glucose.

Many bacteria capable of utilizing methanol as the sole source of energy and carbon exist, such as *Bacillus methanolicus, Methylobacterium extorquens, Methylobacillus glycogenes and Methylobacillus flagellatus.* Methanol-utilizing or methylotrophic bacteria are commonly found in harsh natural environments with poor access to nutrients or inside/in close association with plants. Adaptation to such conditions conveys several properties desirable in industrial fermentation microorganisms, such as the ability to grow quickly in minimal media and resistance to fluctuating nutrient and oxygen supply. A specific example is the ability to store reserve phosphate in the form of insoluble polyphosphate granules, facilitated by a polyphosphate kinase (ppK) enzyme. This allows the organisms to assimilate more phosphate than is required for growth when it is available and utilize the reserve when it is scarce. Members of *Methylobacillus* genus possess many such favourable qualities and are promising candidates for use in industrial methanol fermentations.

However, some environmental adaptations can lead to undesirable phenotypes in an industrial biotechnological context. Programmed cell death and other cell lysis mechanisms are an example of such an adaptation. When nutrients are scarce, the population is too high or as a response to other stressors, it is often beneficial to the population as a whole if the majority of individual bacteria undergo lysis. This releases nutrients to the small subset of surviving cells, allowing them to survive where a larger population could potentially be eliminated completely. Programmed cell death in unicellular organisms is a poorly understood phenomenon that was only recently accepted as real by the scientific community, since it would require a certain degree of "selflessness" from single-cell organisms. While specific triggers are not known, it has been linked to quorum-sensing mechanisms. Such behaviour is highly undesirable in industrial bioreactors, where cell densities should reach extremely high values. In many bioprocesses, most product is formed in the stationary phase, after maximum cell density is reached and carbon starts flowing towards product formation rather than biomass. Thus, mass cell lysis which occurs at early stages of the bioprocess in many organisms, for example *Methylobacillus* spp., significantly reduces achievable product yields and titres.

### Summary of the invention

The object of the present invention is to overcome drawbacks in bioprocesses, especially with respect to mass cell lysis occurring at early stages thereof. This is achieved by the present inventors who have engineered genetically modified bacteria which show resistance to mass cell lysis.

Based on model bacteria of the genus *Methylobacillus,* the present inventors have observed an unexpected complete elimination of sudden mass cell lysis by decreasing the expression and/or activity of an endogenous polypeptide having polyphosphate kinase (PPK) activity. While neither the gene or the encoded protein have ever been linked to cell lysis or programmed cell death in microorganisms before, decreasing (e.g., disrupting) its function in bacteria comprising an endogenous polyphosphate kinase prevented cell lysis under all tested conditions that would otherwise lead to lysis. This significantly improves their usefulness in chemical production, since they can be cultured to higher cell densities and remain metabolically active and produce more product in longer periods of time.

Additionally, the present inventors have observed that the decrease (e.g. disruption) of Acyl-homoserine-lactone (AHL) synthase function prevents lysis under certain conditions (Carbon limitation).

Based on the foregoing finds, the present invention provides in a first aspect a genetically engineered bacterium which has been modified to have a decreased expression and/or activity of an endogenous polypeptide having polyphosphate kinase activity compared to an otherwise identical bacterium that does not carry said modification.

The present invention further provides a method for the production of a biochemical compound comprising cultivating a bacterium of the present invention under suitable culture conditions.

The present invention may be summarized by the following items:
1. A genetically engineered bacterium which has been modified to have a decreased expression and/or activity of an endogenous polypeptide having polyphosphate kinase activity compared to an otherwise identical bacterium that does not carry said modification.
2. The bacterium according to item 1, which has been modified to have a decreased expression of an endogenous polypeptide having polyphosphate kinase activity compared to an otherwise identical bacterium that does not carry said modification.
3. The bacterium according to item 1 or 2, wherein the expression level of the endogenous polypeptide having polyphosphate kinase activity is decreased by at least 50%, such as by at least 60%, at least 65%, at least 70%, at least 75%, at least 80%, at least 85%, at least 90%, at least 95% or at least 100% compared to the otherwise identical bacterium.
4. The bacterium according to any one of item 1 to 3, wherein the endogenous gene encoding said polypeptide having polyphosphate kinase activity has been inactivated.
5. The bacterium according to item 4, wherein the endogenous gene encoding said polypeptide having polyphosphate kinase activity has been inactivated by deletion of part of or the entire gene sequence.
6. The bacterium according to item 3 or 4, wherein the endogenous gene encoding said polypeptide having polyphosphate kinase activity has been inactivated by introducing or expressing in the bacterium a rare-cutting endonuclease able to selectively inactivating by DNA cleavage the endogenous gene encoding said polypeptide.
7. The bacterium according to item 6, wherein said rare-cutting endonuclease is a transcription activator-like effector (TALE) nuclease, meganuclease, zinc-finger nuclease (ZFN), or RNA-guided endonuclease.
8. The bacterium according to item 7, wherein the RNA-guided endonuclease is a catalytically inactive Cas9 protein.
9. The bacterium according to item 8, which comprises (e.g., expresses) a single guide RNA (sgRNA) specifically hybridizing (e.g. binding) under cellular conditions with the genomic DNA encoding said polypeptide.
10. The bacterium according to any one of items 1 to 3, wherein the expression of said endogenous polypeptide having polyphosphate kinase activity is decreased (e.g., inhibited) by transcriptional and/or translational repression of the endogenous gene encoding said polypeptide.
11. The bacterium according to any one of items 1 to 3, wherein the expression of said endogenous polypeptide having polyphosphate kinase activity is decreased (e.g. inhibited) by introducing or expressing in the bacterium an inhibitory nucleic acid molecule that specifically hybridizes (e.g. binds) under cellular conditions with cellular mRNA and/or genomic DNA encoding said polypeptide.
12. The bacterium according to item 11, wherein the inhibitory nucleic acid molecule is an antisense oligonucleotide, ribozyme or interfering RNA (RNAi) molecule.
14. The bacterium according to item 12, wherein the interfering RNA molecule is a micro RNA (miRNA), small interfering RNA (siRNA) or short hairpin RNA (shRNA).
15. The bacterium according to item 1, which has been modified to have a decreased activity of an endogenous polypeptide having polyphosphate kinase activity compared to an otherwise identical microorganism that does not carry said modification.
16. The bacterium according to item 15, wherein the activity of said polypeptide is decreased by at least one active-site mutation resulting in the reduction or loss of activity.
17. The bacterium according to item 16, wherein the at least one active-site mutation is a non-conservative amino acid substitution.
18. The bacterium according to any one of items 1 to 17, which has been further modified to have a decreased expression and/or activity of an endogenous polypeptide having Acyl-homoserine-lactone (AHL) synthase activity compared to an otherwise identical bacterium that does not carry said modification.
19. The bacterium according to any one of items 1 to 18, which has been modified to have a decreased expression of an endogenous polypeptide having Acyl-homoserine-lactone (AHL) synthase activity compared to an otherwise identical bacterium that does not carry said modification.
20. The bacterium according to item 18 or 19, wherein the expression level of the endogenous polypeptide having Acyl-homoserine-lactone (AHL) synthase activity is decreased by at least 50%, such as by at least 60%, at least 65%, at least 70%, at least 75%, at least 80%, at least 85%, at least 90%, at least 95% or at least 100% compared to the otherwise identical bacterium.
21. The bacterium according to any one of item 18 to 20, wherein the endogenous gene encoding said polypeptide having Acyl-homoserine-lactone (AHL) synthase activity has been inactivated.
22. The bacterium according to item 21, wherein the endogenous gene encoding said polypeptide having Acyl-homoserine-lactone (AHL) synthase activity has been inactivated by deletion of part of or the entire gene sequence.
23. The bacterium according to item 21 or 22, wherein the endogenous gene encoding said polypeptide having Acyl-homoserine-lactone (AHL) synthase activity has been inactivated by introducing or expressing in the bacterium a rare-cutting endonuclease able to selectively inactivating by DNA cleavage the endogenous gene encoding said polypeptide.
24. The bacterium according to item 23, wherein said rare-cutting endonuclease is a transcription activator-like effector (TALE) nuclease, meganuclease, zinc-finger nuclease (ZFN), or RNA-guided endonuclease.
25. The bacterium according to item 24, wherein the RNA-guided endonuclease is a catalytically inactive Cas9 protein.
26. The bacterium according to item 25, which comprises (e.g., expresses) a single guide RNA (sgRNA) specifically hybridizing (e.g. binding) under cellular conditions with the genomic DNA encoding said polypeptide.
27. The bacterium according to item 18 or 19, wherein the expression of said endogenous polypeptide having Acyl-homoserine-lactone (AHL) synthase activity is decreased (e.g., inhibited) by transcriptional and/or translational repression of the endogenous gene encoding said polypeptide.
28. The bacterium according to item 18 or 19, wherein the expression of said endogenous polypeptide having Acyl-homoserine-lactone (AHL) synthase activity is decreased (e.g. inhibited) by introducing or expressing in the bacterium an inhibitory nucleic acid molecule that specifically hybridizes (e.g. binds) under cellular conditions with cellular mRNA and/or genomic DNA encoding said polypeptide.
29. The bacterium according to item 28, wherein the inhibitory nucleic acid molecule is an antisense oligonucleotide, ribozyme or interfering RNA (RNAi) molecule.
30. The bacterium according to item 29, wherein the interfering RNA molecule is a micro RNA (miRNA), small interfering RNA (siRNA) or short hairpin RNA (shRNA).
31. The bacterium according to item 18, which has been modified to have a decreased activity of an endogenous polypeptide having Acyl-homoserine-lactone (AHL) synthase activity compared to an otherwise identical microorganism that does not carry said modification.
32. The bacterium according to item 31, wherein the activity of said polypeptide is decreased by at least one active-site mutation resulting in the reduction or loss of activity.
33. The bacterium according to item 32, wherein the at least one active-site mutation is a non-conservative amino acid substitution.
34. The bacterium according to any one of items 1 to 33, which is a mesophilic, methylotrophic bacterium.
35. The bacterium according to any one of items 1 to 34, wherein said bacterium belongs to the genus *Methylobacillus, Methylobacterium or Methylorubrum, preferably Methylobacillus* or *Methylobacterium.*
36. The bacterium according to any one of items 1 to 35, which is selected from *Methylobacillus flagellatus, Methylobacillus glycogenes, Methylobacillus pratensis, Methylobacillus rhizosphaerae, Methylobacillus gramineus, Methylobacillus arboreus, Methylobacillus caricics, Methylobacillus methilovorans, Methylobacillus sp, Methylobacterium extorquens, Methylobacterium organophilum and Methylorubrum extorquens.*
37. The bacterium according to any one of items 1 to 36, which is of the genus *Methylobacillus.*
38. The bacterium according to any one of items 1 to 37, which is selected from *Methylobacillus flagellatus, Methylobacillus glycogenes, Methylobacillus pratensis, Methylobacillus rhizosphaerae, Methylobacillus gramineus, Methylobacillus arboreus, Methylobacillus caricics, Methylobacillus methilovorans and Methylobacillus sp.*
39. The bacterium according to any one of items 1 to 38, wherein said bacterium is *Methylobacillus flagellatus.*
40. The bacterium according to any one of items 1 to 38, wherein said bacterium is *Methylobacillus glycogenes.*
41. The bacterium according to any one of items 37 to 40 which has been modified to have a decreased production of exopolysaccharides (EPS) compared to an otherwise identical bacterium that does not carry said modification.
42. The bacterium according to item 41, which has been modified to have a decreased expression and/or activity of at least one endogenous polypeptide involved in the production of exopolysaccharides (EPS) in said bacterium compared to an otherwise identical bacterium that does not carry said modification.
43. The bacterium according to item 41 or 42, which has been modified to have a decreased expression of at least one endogenous polypeptide involved in the production of exopolysaccharides (EPS) in said bacterium compared to an otherwise identical bacterium that does not carry said modification.
44. The bacterium according to any one of items 41 to 43, which has been modified to have a decrease expression of at least two endogenous polypeptides involved in the production of exopolysaccharides (EPS) in said bacterium compared to an otherwise identical bacterium that does not carry said modification.
45. The bacterium according to any one of items 41 to 44, which has been modified to have a decrease expression of at least three endogenous polypeptides involved in the production of exopolysaccharides (EPS) in said bacterium compared to an otherwise identical bacterium that does not carry said modification.
46. The bacterium according to any one of items 41 to 45, which has been modified to have a decrease expression of at least four endogenous polypeptides involved in the production of exopolysaccharides (EPS) in said bacterium compared to an otherwise identical bacterium that does not carry said modification.
47. The bacterium according to any one of items 41 to 46, which has been modified to have a decrease expression of at least five endogenous polypeptides involved in the production of exopolysaccharides (EPS) in said bacterium compared to an otherwise identical bacterium that does not carry said modification.
48. The bacterium according to any one of items 41 to 47, which has been modified to have a decrease expression of at least six endogenous polypeptides involved in the production of exopolysaccharides (EPS) in said bacterium compared to an otherwise identical bacterium that does not carry said modification.
49. The bacterium according to any one of items 1 to 8, which has been modified to have a decrease expression of all endogenous polypeptides involved in the production of exopolysaccharides (EPS) in said bacterium compared to an otherwise identical bacterium that does not carry said modification.
50. The bacterium according to any one of items 42 to 49, wherein the at least one polypeptide involved in the production of exopolysaccharides (EPS) in said bacterium is selected from the group consisting of a) polypeptides comprising an amino acid sequence set forth in any one of SEQ ID NO: 27 to 64 and b) polypeptides comprising an amino acid sequence having at least 70% sequence identity with any one of SEQ ID NO: 27 to 64 and having the same functional property as the reference polypeptide.
51. The bacterium according to any one of items 42 to 50, wherein the at least one polypeptide involved in the production of exopolysaccharides (EPS) in said bacterium is selected from the group consisting of a) polypeptides comprising an amino acid sequence set forth in any one of SEQ ID NO: 27 to 52 and b) polypeptides comprising an amino acid sequence having at least 70% sequence identity with any one of SEQ ID NO: 27 to 52 and having the same functional property as the reference polypeptide.
52. The bacterium according to any one of items 42 to 50, wherein the at least one polypeptide involved in the production of exopolysaccharides (EPS) in said bacterium is selected from the group consisting of a) polypeptides comprising an amino acid sequence set forth in any one of SEQ ID NO: 53 to 64 and b) polypeptides comprising an amino acid sequence having at least 70% sequence identity with any one of SEQ ID NO: 53 to 64 and having the same functional property as the reference polypeptide.
53. The bacterium according to any one of items 42 to 52, wherein the at least one polypeptide involved in the production of exopolysaccharides (EPS) in said bacterium is selected from the group consisting of a) polypeptides comprising an amino acid sequence set forth in any one of SEQ ID NO: 65 to 112 and b) polypeptides comprising an amino acid sequence having at least 70% sequence identity with any one of SEQ ID NO: 65 to 112 and having the same functional property as the reference polypeptide.
54. The bacterium according to any one of items 42 to 53, wherein the at least one polypeptide involved in the production of exopolysaccharides (EPS) in said bacterium is selected from the group consisting of a) polypeptides comprising an amino acid sequence set forth in any one of SEQ ID NO: 65 to 86 and b) polypeptides comprising an amino acid sequence having at least 70% sequence identity with any one of SEQ ID NO: 65 to 86 and having the same functional property as the reference polypeptide.
55. The bacterium according to any one of items 42 to 53, wherein the at least one polypeptide involved in the production of exopolysaccharides (EPS) in said bacterium is selected from the group consisting of a) polypeptides comprising an amino acid sequence set forth in any one of SEQ ID NO: 87 to 112 and b) polypeptides comprising an amino acid sequence having at least 70% sequence identity with any one of SEQ ID NO: 87 to 112 and having the same functional property as the reference polypeptide.
56. The bacterium according to any one of items 42 to 55, wherein the at least one polypeptide involved in the production of exopolysaccharides (EPS) in said bacterium is selected from the group consisting a polypeptide having peptidyl-prolyl cis-trans isomerase activity, a polypeptide capable of acting as a polysaccharide exporter; a polypeptide acting as a chain length determinant protein; and a polypeptide having protein-tyrosine kinase activity.
57. The bacterium according to item 56, wherein the polypeptide having peptidyl-prolyl cis-trans isomerase activity comprises an amino acid sequence having at least 70% sequence identity to SEQ ID NO: 32, the polypeptide capable of acting as a polysaccharide exporter comprises an amino acid sequence having at least 70% sequence identity to SEQ ID NO: 33, the polypeptide acting as a chain length determinant protein comprises an amino acid sequence having at least 70% sequence identity to SEQ ID NO: 34 and the polypeptide having protein-tyrosine kinase activity comprises an amino acid sequence having at least 70% sequence identity to SEQ ID NO: 35.
58. The bacterium according to item 56, wherein the polypeptide having peptidyl-prolyl cis-trans isomerase activity comprises an amino acid sequence having at least 70% sequence identity to SEQ ID NO: 68, the polypeptide capable of acting as a polysaccharide exporter comprises an amino acid sequence having at least 70% sequence identity to SEQ ID NO: 69, the polypeptide acting as a chain length determinant protein comprises an amino acid sequence having at least 70% sequence identity to SEQ ID NO: 70 and the polypeptide having protein-tyrosine kinase activity comprises an amino acid sequence having at least 70% sequence identity to SEQ ID NO: 71.
59. The bacterium according to any one of items 42 to 58, wherein the expression of the at least one polypeptide involved in the production of exopolysaccharides (EPS) in said bacterium is decreased by at least 50%, such as by at least 60%, at least 65%, at least 70%, at least 75%, at least 80%, at least 85%, at least 90%, at least 95% or at least 100% compared to the otherwise identical bacterium.
60. The bacterium according to any one of items 42 to 59, wherein the expression of the at least one polypeptide involved in the production of exopolysaccharides (EPS) in said bacterium is abolished compared to the otherwise identical bacterium.
61. The bacterium according to any one of items 42 to 60, wherein the endogenous gene/s encoding said polypeptide/s involved in the production of exopolysaccharides (EPS) in said bacterium has/have been inactivated.
62. The bacterium according to any one of items 42 to 61, wherein the endogenous gene/s encoding said polypeptides/s involved in the production of exopolysaccharides (EPS) in said bacterium has/have been inactivated by deletion of part of or the entire gene sequence.
63. The bacterium according to any one of items 42 to 62, wherein the endogenous gene/s encoding said polypeptide/s involved in the production of exopolysaccharides (EPS) in said bacterium has/have been inactivated by introducing or expressing in the bacterium a respective rare-cutting endonuclease able to selectively inactivating by DNA cleavage the endogenous gene encoding said polypeptide.
64. The bacterium according to item 63, wherein said rare-cutting endonucleases are selected from the group consisting of a transcription activator-like effector (TALE) nuclease, meganuclease, zing-finger nuclease (ZFN) and RNA-guided endonuclease.
65. The bacterium according to item 64, wherein the RNA-guided endonuclease is a catalytically inactive Cas9 protein.
66. The bacterium according to item 65, which comprises (e.g., expresses) at least one single guide RNA (sgRNA) specifically hybridizing (e.g. binding) under cellular conditions with the genomic DNA encoding said enzyme(s).
67. The bacterium according to any one of items 42 to 61, wherein the expression of the polypeptide(s) involved in the production of exopolysaccharides (EPS) in said bacterium is decreased (e.g., inhibited) by transcriptional and/or translational repression of the endogenous gene(s) encoding said polypeptide(s).
68. The bacterium according to any one of items 42 to 61, wherein the expression of the polypeptide(s) involved in the production of exopolysaccharides (EPS) in said bacterium is decreased (e.g., inhibited) by introducing or expressing in the bacterium at least one inhibitory nucleic acid molecule that specifically hybridizes (e.g. binds) under cellular conditions with cellular mRNA and/or genomic DNA encoding said polypeptide(s).
69. The bacterium according to item 68, wherein the inhibitory nucleic acid molecule is an antisense oligonucleotide, ribozyme or interfering RNA (RNAi) molecule.
70. The bacterium according to item 69, wherein the interfering RNA molecule is a micro RNA (miRNA), small interfering RNA (siRNA) or short hairpin RNA (shRNA).
71. The bacterium according to any one of items 42 to 70, wherein the endogenous polypeptide or polypeptides involved in the production of exopolysaccharides (EPS) in said bacterium is/are encoded by a gene or genes included in a first EPS gene cluster and/or second EPS gene cluster.
72. The bacterium according to item 71, wherein the first EPS gene cluster includes genes defined by or orthologous to the open reading frames (ORFs) found in SEQ ID NO: 200 or 203.
73. The bacterium according to item 71 or 72, wherein the first EPS gene cluster includes the genes *epsD, epsE, epsF, epsG, epsB, epsL, epsH, epsl, epsJ* and *epsS,* or orthologs thereof.
74. The bacterium according to any one of items 71 to 73, wherein the first EPS gene cluster includes the genes *epsD, epsE, epsF* and *epsG,* or orthologs thereof.
75. The bacterium according to any one of items 71 to 74, wherein the first EPS gene cluster includes a nucleotide sequence which has at least 50% sequence identity with the nucleotide sequence of SEQ ID NO: 200 or 203.
76. The bacterium according to any one of items 71 to 74, wherein the first EPS gene cluster includes a nucleotide sequence which has at least 50% sequence identity with the nucleotide sequence of SEQ ID NO: 200.
77. The bacterium according to any one of items 71 to 76, wherein at least one gene in said first EPS gene cluster is inactivated, e.g., by deletion of part of or the entire gene sequence.
78. The bacterium according to any one of items 71 to 77, wherein at least one gene selected from *epsD, epsE, epsF, epsG, epsB, epsL, epsH, epsl, epsJ* and *epsS,* or ortholog thereof, is inactivated, e.g., by deletion of part of or the entire gene sequence.
79. The bacterium according to any one of items 71 to 78, wherein at least one gene selected from *epsD, epsE, epsF* and *epsG,* or ortholog thereof, is inactivated, e.g., by deletion of part of or the entire gene sequence.
80. The bacterium according to any one of items 71 to 79, wherein the genes *epsD, epsE, epsF and epsG,* or orthologs thereof, are inactivated, e.g., by deletion of part of or the entire gene sequence.
81. The bacterium according to item 79 or 80, wherein the gene *epsD* encodes a polypeptide comprising an amino acid sequence having at least 70% sequence identity to SEQ ID NO: 32, wherein the gene epsE encodes a polypeptide comprising an amino acid sequence having at least 70% sequence identity to SEQ ID NO: 33, wherein the gene epsF encodes a polypeptide comprising an amino acid sequence having at least 70% sequence identity to SEQ ID NO: 34, and wherein the gene epsG encodes a polypeptide comprising an amino acid sequence having at least 70% sequence identity to SEQ ID NO: 35.
82. The bacterium according to item 81, wherein the gene *epsD* comprises a nucleotide sequence having at least 70% sequence identity with SEQ ID NO: 118, the gene *epsE* comprises a nucleotide sequence having at least 70% sequence identity with SEQ ID NO: 119, the gene *epsF* comprises an nucleotide sequence having at least 70% sequence identity with SEQ ID NO: 120, and the gene *epsG* comprises an nucleotide sequence having at least 70% sequence identity with SEQ ID NO: 121.
83. The bacterium according to item 79 or 80, wherein the gene *epsD* encodes a polypeptide comprising an amino acid sequence having at least 70% sequence identity to SEQ ID NO: 68, wherein the gene *epsE* encodes a polypeptide comprising an amino acid sequence having at least 70% sequence identity to SEQ ID NO: 69, wherein the gene *epsF* encodes a polypeptide comprising an amino acid sequence having at least 70% sequence identity to SEQ ID NO: 70, and wherein the gene *epsG* encodes a polypeptide comprising an amino acid sequence having at least 70% sequence identity to SEQ ID NO: 71.
84. The bacterium according to item 83, wherein the *epsD* gene comprises a nucleotide sequence having at least 70% sequence identity with SEQ ID NO: 154, the gene *epsE* comprises a nucleotide sequence having at least 70% sequence identity with SEQ ID NO: 155, the gene *epsF* comprises an nucleotide sequence having at least 70% sequence identity with SEQ ID NO: 156, and the gene *epsG* comprises an nucleotide sequence having at least 70% sequence identity with SEQ ID NO: 157.
85. The bacterium according to any one of items 71 to 84, wherein the first EPS gene cluster is inactivated.
86. The bacterium according to any one of items 71 to 85, wherein the first EPS gene cluster is inactivated by deletion of part of or the entire sequence of said cluster.
87. The bacterium according to any one of items 71 to 85, wherein the first EPS gene cluster is inactivated by modification of (e.g., by introducing at least one mutation in) the promoter and/or ribosome binding site region resulting in the lack of gene expression.
88. The bacterium according to any one of items 71 to 87, wherein the second EPS gene cluster includes genes defined by or orthologous to the open reading frames (ORFs) found in SEQ ID NO: 201 or 205.
89. The bacterium according to any one of items 71 to 88, wherein the second EPS gene cluster includes a nucleotide sequence which has at least 50% sequence identity with the nucleotide sequence of 201 or 205.
90. The bacterium according to any one of items 71 to 88, wherein the second EPS gene cluster includes a nucleotide sequence which has at least 50% sequence identity with the nucleotide sequence of 201.
91. The bacterium according to any one of items 71 to 90, wherein at least one gene in said second EPS gene cluster is inactivated, e.g., by deletion of part of or the entire gene sequence.
92. The bacterium according to any one of items 71 to 91, wherein the second EPS gene cluster is inactivated.
93. The bacterium according to any one of items 71 to 92, wherein the second EPS gene cluster is inactivated by deletion of part of or the entire sequence of said cluster.
94. The bacterium according to any one of items 71 to 92, wherein the second EPS gene cluster is inactivated by modification of (e.g., by introducing at least one mutation in) the promoter and/or ribosome binding site region resulting in the lack of gene expression.
95. The bacterium according to any one of items 40 to 92, wherein all endogenous genes encoding enzymes involved in the production of exopolysaccharides (EPS) in said bacterium are inactivated, e.g., deleted.
96. Method for producing a biochemical comprising cultivating a bacterium according to any one of items 1 to 95 under suitable culture conditions.
97. The method according to item 96 comprising cultivating a bacterium according to any one of items 40 to 95 under suitable culture conditions in a culture medium comprising a reduced one-carbon compound, such as methanol, or a multi-carbon compound that contains no carbon-carbon bonds, such as dimethyl ether and dimethylamine.
98. The method according to item 97, wherein the culture medium comprises methanol.
99. The method according to any one of items 96 to 98, wherein the cultivation is performed in a bioreactor.
100. The method according to any one of items 96 to 99, wherein the biochemical compound is selected from organic acids, amino acids, fatty acids and derivatives thereof.
101. Method for producing biomass comprising cultivating a bacterium according to any one of items 1 to 95 under suitable culture conditions.

### Brief description of the figures

**Figure 1****:** Fluorescent image of *Methylobacillus flagellatus* cells stained with DAPI under 400x magnification, wild-type cells on the left and ppK deletion cells on the right. Arrows point to polyphosphate granules, stained yellow by the DAPI stain.
**Figure 2****:** Biomass-production bioprocess plots of fermentations using wild-type (top) and ppK deficient *Methylobacillus flagellatus* (bottom).
**Figure 3****:** GABA-production bioprocess plots of fermentations using wild-type (top) and ppK deficient *Methylobacillus flagellatus* (bottom) expressing glutamate dehydrogenase enzymes.
**Figure 4****:** Screen capture of the antiSMASH algorithm output, with known N-acyl homoserine lactone production clusters from other organisms
**Figure 5****:** Biomass-production bioprocess plots of fermentations using wild-type (top) and AHL-synthase deficient *Methylobacillus flagellatus* (bottom).
**Figure 6****:** Sugar content of hydrolyzed *M. flagellatus* shake-flask supernatant. Dark gray bars represent the total glucose in mg/L and the light gray bars represent total monomeric sugars. Error bars are the standard deviation of technical replicates.
**Figure 7****:** Measured viscosity of bioreactor broth produced by different strains of *Methylobacillus flagellatus.*
**Figure 8****:** Photo of wild type M. flagellatus and M. glycogenes cultures after 24 hours of incubation. Wild type ABME 5 and 6 on the left, followed by a single-cluster disruption in *M. flagellatus* and finally a double disruption.
**Figure 9****:** Plot of antifoam addition (y axis) during two representative bioreactor fermentations of ABME 6 and ABME 131 in relation to fermentation time (x axis).
**Figure 10****:** Phase-contrast microscopy images of bioreactor fermentation broth of wild type *Methylobacillus flagellatus* (left) vs the double-disruption strain ABME 131 (right).
**Figure 11****:** Improved growth of the EPS free ABME131 compared to the parent strain.

The present invention is now described in more detail below.

### Detailed description of the invention

Unless specifically defined herein, all technical and scientific terms used have the same meaning as commonly understood by a skilled artisan in the fields of biochemistry, genetics, and microbiology.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, and recombinant DNA, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory).

### Bacterium of the invention

As indicated above, the present invention is based on the unexpected and surprising finding that certain drawbacks in bioprocesses, notably mass cell lysis, can be overcome by decreasing the expression and/or activity of an endogenous polypeptide having polyphosphate kinase activity in bacteria.

The present invention thus provides in a first aspect a genetically engineered bacterium which has been modified to have a decreased expression and/or activity of an endogenous polypeptide having polyphosphate kinase activity compared to an otherwise identical bacterium that does not carry said modification.

A "polypeptide having polyphosphate kinase activity" is a polypeptide that catalyzes the reaction: ATP + (phosphate)ₙ <=> ADP + (phosphate)ₙ₊₁ (EC 2.7.4.1). Polyphosphate kinase (PPK), which is encoded by the *ppk* gene, is highly conserved in many bacteria, including methylotrophs such as *Methylobacillus flagellatus* and *Methylobacillus glycogenes,* and plays a crucial role in the ability of bacteria to adapt to nutritional stringencies and environmental stresses. Non-limiting examples of an endogenous polypeptide having polyphosphate kinase activity are provided in SEQ ID NOs: 1, 3, 5, 7, 9 and 11.

According to some embodiments, the endogenous polypeptide having polyphosphate kinase activity comprises an amino acid sequence having at least 70%, such as at least 75%, sequence identity with the amino acid sequence set forth in SEQ ID NO: 1. According to some embodiments, the endogenous polypeptide having polyphosphate kinase activity comprises an amino acid sequence having at least 80%, such as at least 85%, sequence identity with the amino acid sequence set forth in SEQ ID NO: 1. According to some embodiments, the endogenous polypeptide having polyphosphate kinase activity comprises an amino acid sequence having at least 90%, such as at least 95%, sequence identity with the amino acid sequence set forth in SEQ ID NO: 1.

According to some embodiments, the endogenous gene encoding the polypeptide having polyphosphate kinase activity comprises a nucleic acid sequence having at least 70%, such as at least 75%, , sequence identity with the nucleic acid sequence set forth in SEQ ID NO: 2. According to some embodiments, the endogenous gene encoding the polypeptide having polyphosphate kinase activity comprises a nucleic acid sequence having at least 80%, such as at least 85%, sequence identity with the nucleic acid sequence set forth in SEQ ID NO: 2. According to some embodiments, the endogenous gene encoding the polypeptide having polyphosphate kinase activity comprises a nucleic acid sequence having at least 90%, such as at least 95%, sequence identity with the nucleic acid sequence set forth in SEQ ID NO: 2.

According to some embodiments, the endogenous polypeptide having polyphosphate kinase activity comprises an amino acid sequence having at least 70%, such as at least 75%, sequence identity with the amino acid sequence set forth in SEQ ID NO: 3. According to some embodiments, the endogenous polypeptide having polyphosphate kinase activity comprises an amino acid sequence having at least 80%, such as at least 85%, sequence identity with the amino acid sequence set forth in SEQ ID NO: 3. According to some embodiments, the endogenous polypeptide having polyphosphate kinase activity comprises an amino acid sequence having at least at least 90%, such as at least 95%, sequence identity with the amino acid sequence set forth in SEQ ID NO: 3.

According to some embodiments, the endogenous gene encoding the polypeptide having polyphosphate kinase activity comprises a nucleic acid sequence having at least 70%, such as at least 75%, sequence identity with the nucleic acid sequence set forth in SEQ ID NO: 4. According to some embodiments, the endogenous gene encoding the polypeptide having polyphosphate kinase activity comprises a nucleic acid sequence having at least at least 80%, such as at least 85%, sequence identity with the nucleic acid sequence set forth in SEQ ID NO: 4. According to some embodiments, the endogenous gene encoding the polypeptide having polyphosphate kinase activity comprises a nucleic acid sequence having at least at least 90%, such as at least 95%, sequence identity with the nucleic acid sequence set forth in SEQ ID NO: 4.

According to some embodiments, the endogenous polypeptide having polyphosphate kinase activity comprises an amino acid sequence having at least 70%, such as at least 75%, sequence identity with the amino acid sequence set forth in SEQ ID NO: 5. According to some embodiments, the endogenous polypeptide having polyphosphate kinase activity comprises an amino acid sequence having at least 80%, such as at least 85%, sequence identity with the amino acid sequence set forth in SEQ ID NO: 5. According to some embodiments, the endogenous polypeptide having polyphosphate kinase activity comprises an amino acid sequence having at least at least 90%, such as at least 95%, sequence identity with the amino acid sequence set forth in SEQ ID NO: 5. According to some embodiments, the endogenous gene encoding the polypeptide having polyphosphate kinase activity comprises a nucleic acid sequence having at least 70%, such as at least 75%, sequence identity with the nucleic acid sequence set forth in SEQ ID NO: 6. According to some embodiments, the endogenous gene encoding the polypeptide having polyphosphate kinase activity comprises a nucleic acid sequence having at least 80%, such as at least 85%, sequence identity with the nucleic acid sequence set forth in SEQ ID NO: 6. According to some embodiments, the endogenous gene encoding the polypeptide having polyphosphate kinase activity comprises a nucleic acid sequence having at least 90%, such as at least 95%, sequence identity with the nucleic acid sequence set forth in SEQ ID NO: 6.

According to some embodiments, the endogenous polypeptide having polyphosphate kinase activity comprises an amino acid sequence having at least 70%, such as at least 75%, sequence identity with the amino acid sequence set forth in SEQ ID NO: 7. According to some embodiments, the endogenous polypeptide having polyphosphate kinase activity comprises an amino acid sequence having at least 80%, such as at least 85%, sequence identity with the amino acid sequence set forth in SEQ ID NO: 7. According to some embodiments, the endogenous polypeptide having polyphosphate kinase activity comprises an amino acid sequence having at least 90%, such as at least 95%, sequence identity with the amino acid sequence set forth in SEQ ID NO: 7.

According to some embodiments, the endogenous gene encoding the polypeptide having polyphosphate kinase activity comprises a nucleic acid sequence having at least 70%, such as at least 75%, sequence identity with the nucleic acid sequence set forth in SEQ ID NO: 8. According to some embodiments, the endogenous gene encoding the polypeptide having polyphosphate kinase activity comprises a nucleic acid sequence having at least 80%, such as at least 85%, sequence identity with the nucleic acid sequence set forth in SEQ ID NO: 8. According to some embodiments, the endogenous gene encoding the polypeptide having polyphosphate kinase activity comprises a nucleic acid sequence having at least 90%, such as at least 95%, sequence identity with the nucleic acid sequence set forth in SEQ ID NO: 8.

According to some embodiments, the endogenous polypeptide having polyphosphate kinase activity comprises an amino acid sequence having at least 70%, such as at least 75%, sequence identity with the amino acid sequence set forth in SEQ ID NO: 9. According to some embodiments, the endogenous polypeptide having polyphosphate kinase activity comprises an amino acid sequence having at least 80%, such as at least 85%, sequence identity with the amino acid sequence set forth in SEQ ID NO: 9. According to some embodiments, the endogenous polypeptide having polyphosphate kinase activity comprises an amino acid sequence having at least 90%, such as at least 95%, sequence identity with the amino acid sequence set forth in SEQ ID NO: 9.

According to some embodiments, the endogenous gene encoding the polypeptide having polyphosphate kinase activity comprises a nucleic acid sequence having at least 70%, such as at least 75%, sequence identity with the nucleic acid sequence set forth in SEQ ID NO: 10. According to some embodiments, the endogenous gene encoding the polypeptide having polyphosphate kinase activity comprises a nucleic acid sequence having at least 80%, such as at least 85%, sequence identity with the nucleic acid sequence set forth in SEQ ID NO: 10. According to some embodiments, the endogenous gene encoding the polypeptide having polyphosphate kinase activity comprises a nucleic acid sequence having at least 90%, such as at least 95%, sequence identity with the nucleic acid sequence set forth in SEQ ID NO: 10.

According to some embodiments, the endogenous polypeptide having polyphosphate kinase activity comprises an amino acid sequence having at least 70%, such as at least 75%, sequence identity with the amino acid sequence set forth in SEQ ID NO: 11. According to some embodiments, the endogenous polypeptide having polyphosphate kinase activity comprises an amino acid sequence having at least 80%, such as at least 85%, sequence identity with the amino acid sequence set forth in SEQ ID NO: 11. According to some embodiments, the endogenous polypeptide having polyphosphate kinase activity comprises an amino acid sequence having at least 90%, such as at least 95%, sequence identity with the amino acid sequence set forth in SEQ ID NO: 11.

According to some embodiments, the endogenous gene encoding the polypeptide having polyphosphate kinase activity comprises a nucleic acid sequence having at least 70%, such as at least 75%, sequence identity with the nucleic acid sequence set forth in SEQ ID NO: 12. According to some embodiments, the endogenous gene encoding the polypeptide having polyphosphate kinase activity comprises a nucleic acid sequence having at least 80%, such as at least 85%, sequence identity with the nucleic acid sequence set forth in SEQ ID NO: 12. According to some embodiments, the endogenous gene encoding the polypeptide having polyphosphate kinase activity comprises a nucleic acid sequence having at least 90%, such as at least 95%, sequence identity with the nucleic acid sequence set forth in SEQ ID NO: 12.

According to some embodiments, the bacterium of the invention may be modified to have a decreased expression of an endogenous polypeptide having polyphosphate kinase activity compared to an otherwise identical bacterium that does not carry said modification.

According to some embodiments, the bacterium of the invention may be modified to have a decreased expression of an endogenous polypeptide having polyphosphate kinase activity compared to an otherwise identical bacterium that does not carry said modification. The expression level of the endogenous polypeptide having polyphosphate kinase activity may, for example, be decreased by at least 50%, such as by at least 60%, at least 65%, at least 70%, at least 75%, at least 80%, at least 85%, at least 90%, at least 95% or at least 100% compared to the otherwise identical bacterium.

According to some embodiments, the bacterium of the invention may be modified to have a decreased expression level of the endogenous gene encoding said endogenous polypeptide having polyphosphate kinase activity compared to an otherwise identical bacterium that does not carry said modification. The expression level of the endogenous gene may, for example, be decreased by at least 50%, such as by at least 60%, at least 65%, at least 70%, at least 75%, at least 80%, at least 85%, at least 90%, at least 95% or at least 100% compared to the otherwise identical bacterium.

According to some embodiments, the endogenous gene encoding said polypeptide having polyphosphate kinase activity has been inactivated, such as by deletion of part of or the entire gene sequence.

According to some embodiments, the endogenous gene encoding said polypeptide having polyphosphate kinase activity has been inactivated by introducing or expressing in the microorganism a rare-cutting endonuclease able to selectively inactivate by DNA cleavage, preferably by a double-strand break, the endogenous gene encoding said enzyme. A rare-cutting endonuclease to be used in accordance with the present invention to inactivate the endogenous gene may, for instance, be a transcription activator-like effector (TALE) nuclease, meganuclease, zinc-finger nuclease (ZFN), or RNA-guided endonuclease.

One way to inactivate the endogenous gene encoding said polypeptide having polyphosphate kinase activity is to use the CRISPRi system. The CRISPRi system was developed as a tool for targeted repression of gene expression or for blocking targeted locations on the genome. The CRISPRi system consists of the catalytically inactive, "dead" Cas9 protein (dCas9) and a guide RNA that defines the binding site for the dCas9 to DNA.

Thus, according to some embodiments, the endogenous gene encoding said polypeptide having polyphosphate kinase activity is inactivated by introducing or expressing in the bacterium an RNA-guided endonuclease, such as a catalytically inactive Cas9 protein, and a single guide RNA (sgRNA) specifically hybridizing (e.g. binding) under cellular conditions with the genomic DNA encoding a said polypeptide.

According to some embodiments, the expression of said endogenous polypeptide having polyphosphate kinase activity is decreased by way of inhibition.

Inhibition of the expression of the said endogenous polypeptide may be achieved by any suitable means known in the art. For example, the expression may be inhibited by gene silencing techniques involving the use of inhibitory nucleic acid molecules, such as antisense oligonucleotides, ribozymes, or interfering RNA (RNAi) molecules, such as microRNA (miRNA), small interfering RNA (siRNA), or short hairpin RNA (shRNA).

According to some embodiments, the expression of said endogenous polypeptide having polyphosphate kinase activity is decreased (e.g., inhibited) by transcriptional and/or translational repression of the endogenous gene encoding said polypeptide.

According to some embodiments, the expression of endogenous polypeptide having polyphosphate kinase activity is inhibited by introducing or expressing in the bacterium an inhibitory nucleic acid molecule. For example, the inhibitory nucleic acid molecule may be introduced by way of an exogenous nucleic acid molecule comprising a nucleotide sequence encoding said inhibitory nucleic acid molecule operably linked to a promoter, such as an inducible promoter, that is functional in the bacterium to cause the production of said inhibitory nucleic acid molecule. Suitably, the inhibitory nucleic acid molecule is one that specifically hybridizes (e.g. binds) under cellular conditions with cellular mRNA and/or genomic DNA encoding the endogenous polypetide. Depending on the target, transcription of the encoding genomic DNA and/or translation of the encoding mRNA is/are inhibited.

According to some embodiments, the inhibitory nucleic acid molecule is an antisense oligonucleotide, ribozyme, or interfering RNA (RNAi) molecule. Preferably, such nucleic acid molecule comprises at least 10 consecutive nucleotides of the complement of the cellular mRNA and/or genomic DNA encoding the polypeptide or enzyme of interest (e.g., the cellular mRNA and/or genomic DNA encoding the polypeptide.

According to some embodiments, the inhibitory nucleic acid is an antisense oligonucleotide. Such antisense oligonucleotide is a nucleic acid molecule (either DNA or RNA), which specifically hybridizes (e.g. binds) under cellular conditions with the cellular mRNA and/or genomic DNA encoding the polypeptide.

According to some embodiments, the inhibitory nucleic acid molecule is a ribozyme, such as a hammerhead ribozyme. A ribozyme molecule is designed to catalytically cleave the mRNA transcript to prevent translation of the polypeptide.

According to some embodiments, the inhibitory nucleic acid molecule is an interfering RNA (RNAi) molecule. RNA interference is a biological process in which RNA molecules inhibit expression, typically destroying specific mRNA. Exemplary types of RNAi molecules include microRNA (miRNA), small interfering RNA (siRNA), and short hairpin RNA (shRNA). According to some embodiments, the RNAi molecule is a miRNA. According to some embodiments, the RNAi molecule is a siRNA. According to some embodiments, the RNAi molecule is an shRNA.

According to some embodiments, the bacterium of the invention has been modified to have a decreased activity of an endogenous polypeptide having polyphosphate kinase activity compared to an otherwise identical bacterium that does not carry said modification.

A decrease of the activity of the polypeptide having polyphosphate kinase activity may be achieved by any suitable means known in the art. For example, the activity may be decreased by introducing one or more mutations in the active site of the polypeptide resulting in the reduction or loss of activity. Thus, according to some embodiments, the activity of the endogenous polypeptide having polyphosphate kinase activity is decreased by at least one active-site mutation resulting in the reduction or loss of activity. At least one active-site mutation may, for example, be at least one non-conservative amino acid substitution.

By way of example, if the activity of the endogenous the polypeptide having polyphosphate kinase activity is to be decreased in *Methylobacillus flagellatus,* the at least one active-site mutation may occur at any one of positions R379, S384, F492, P511, R568, R625, Q679, H439, and H458 in the amino acid sequence set forth in SEQ ID NO: 1 which form part of the active site. In case of orthologous polypeptides having polyphosphate kinase activity, the at least one active-site mutation may be at a position which corresponds to any one of positions R379, S384, F492, P511, R568, R625, Q678, H439, and H458 in the amino acid sequence set forth in SEQ ID NO: 1.

By way of another example, if the activity of the endogenous the polypeptide having polyphosphate kinase activity is to be decreased in *Methylobacillus glycogenes,* the at least one active-site mutation may occur at any one of positions R79, S84, F192, P211, R268, R325, Q378, H139, in the amino acid sequence set forth in SEQ ID NO: 3, which form part of the active site. In case of orthologous polypeptides having polyphosphate kinase activity, the at least one active-site mutation may be at a position which corresponds to any one of positions R79, S84, F192, P211, R268, R325, Q378, H139, H158 in the amino acid sequence set forth in SEQ ID NO: 3.

By way of another example, if the activity of the endogenous the polypeptide having polyphosphate kinase activity is to be decreased in *Methylobacillus rhizosphaerae,* the at least one active-site mutation may occur at any one of positions R379, S384, F492, P511, R568, R625, Q678, H439 and H458 in the amino acid sequence set forth in SEQ ID NO: 5, which form part of the active site. In case of orthologous polypeptides having polyphosphate kinase activity, the at least one active-site mutation may be at a position which corresponds to any one of positions R379, S384, F492, P511, R568, R625, Q678, H439 and H458 in the amino acid sequence set forth in SEQ ID NO: 5.

By way of another example, if the activity of the endogenous the polypeptide having polyphosphate kinase activity is to be decreased in *Methylobacterium organophilum,* the at least one active-site mutation may occur at any one of positions R392, S397, F505, P524, R581, R643, H452 and H471 in the amino acid sequence set forth in SEQ ID NO: 7, which form part of the active site. In case of orthologous polypeptides having polyphosphate kinase activity, the at least one active-site mutation may be at a position which corresponds to any one of positions R392, S397, F505, P524, R581, R643, H452 and H471 in the amino acid sequence set forth in SEQ ID NO: 7.

By way of another example, if the activity of the endogenous the polypeptide having polyphosphate kinase activity is to be decreased in *Methylorubrum extorquens,* the at least one active-site mutation may occur at any one of positions R451, S456, F564, P583, R640, R702, H511 and H530 in the amino acid sequence set forth in SEQ ID NO: 9, which form part of the active site. In case of orthologous polypeptides having polyphosphate kinase activity, the at least one active-site mutation may be at a position which corresponds to any one of positions R451, S456, F564, P583, R640, R702, H511 and H530 in the amino acid sequence set forth in SEQ ID NO: 9.

By way of another example, if the activity of the endogenous the polypeptide having polyphosphate kinase activity is to be decreased in *Escherichia coli,* the at least one active-site mutation may occur at any one of positions R375, S380, F488, P507, R564, R621, Q674, H435 and H454 in the amino acid sequence set forth in SEQ ID NO: 11, which form part of the active site. In case of orthologous polypeptides having polyphosphate kinase activity, the at least one active-site mutation may be at a position which corresponds to any one of positions R375, S380, F488, P507, R564, R621, Q674, H435 and H454 in the amino acid sequence set forth in SEQ ID NO: 11.

The resistance of such bacterium against cell lysis may be further improved, especially under certain conditions such as carbon limitation, by decreasing the expression and/or activity of an endogenous polypeptide having Acyl-homoserine-lactone (AHL) synthase activity.

Thus, according to some embodiments, the bacterium of the present invention may be further modified to have a decreased expression and/or activity of an endogenous polypeptide having Acyl-homoserine-lactone (AHL) synthase activity compared to an otherwise identical bacterium that does not carry said modification.

A "polypeptide having Acyl-homoserine-lactone (AHL) synthase activity" is a polypeptide that catalyzes the reaction: An acyl-[acyl-carrier-protein] + S-adenosyl-L-methionine <=> [acyl-carrier-protein] + S-methyl-5'-thioadenosine + an N-acyl-L-homoserine lactone (EC 2.3.1.184). Acyl-homoserine lactones (AHLs) are small signaling molecules used by many Gram-negative bacteria for coordinating their behavior as a function of their population density. This process, based on the biosynthesis and the sensing of such molecular signals, and referred to as Quorum Sensing (QS), regulates various gene expressions, including growth, virulence, biofilms formation, and toxin production. Non-limiting examples of an endogenous polypeptide having Acyl-homoserine-lactone (AHL) synthase activity are provided in SEQ ID NOs: 13, 15, 17, 19, 21, 23 and 25.

According to some embodiments, the endogenous polypeptide having Acyl-homoserine-lactone (AHL) synthase activity comprises an amino acid sequence having at least 70%, such as at least 75%, sequence identity with the amino acid sequence set forth in SEQ ID NO: 13. According to some embodiments, the endogenous polypeptide having Acyl-homoserine-lactone (AHL) synthase activity comprises an amino acid sequence having at least 80%, such as at least 85%, sequence identity with the amino acid sequence set forth in SEQ ID NO: 13. According to some embodiments, the endogenous polypeptide having Acyl-homoserine-lactone (AHL) synthase activity comprises an amino acid sequence having at least 90%, such as at least 95%, sequence identity with the amino acid sequence set forth in SEQ ID NO: 13.

According to some embodiments, the endogenous gene encoding the polypeptide having Acyl-homoserine-lactone (AHL) synthase activity comprises a nucleic acid sequence having at least 70%, such as at least 75%, sequence identity with the nucleic acid sequence set forth in SEQ ID NO: 14. According to some embodiments, the endogenous gene encoding the polypeptide having Acyl-homoserine-lactone (AHL) synthase activity comprises a nucleic acid sequence having at least 80%, such as at least 85%, sequence identity with the nucleic acid sequence set forth in SEQ ID NO: 14. According to some embodiments, the endogenous gene encoding the polypeptide having Acyl-homoserine-lactone (AHL) synthase activity comprises a nucleic acid sequence having at least 90%, such as at least 95%, sequence identity with the nucleic acid sequence set forth in SEQ ID NO: 14.

According to some embodiments, the endogenous polypeptide having Acyl-homoserine-lactone (AHL) synthase activity comprises an amino acid sequence having at least 70%, such as at least 75%, sequence identity with the amino acid sequence set forth in SEQ ID NO: 15. According to some embodiments, the endogenous polypeptide having Acyl-homoserine-lactone (AHL) synthase activity comprises an amino acid sequence having at least 80%, such as at least 85%, sequence identity with the amino acid sequence set forth in SEQ ID NO: 15. According to some embodiments, the endogenous polypeptide having Acyl-homoserine-lactone (AHL) synthase activity comprises an amino acid sequence having at least 90%, such as at least 95%, sequence identity with the amino acid sequence set forth in SEQ ID NO: 15.

According to some embodiments, the endogenous gene encoding the polypeptide having Acyl-homoserine-lactone (AHL) synthase activity comprises a nucleic acid sequence having at least 70%, such as at least 75%, sequence identity with the nucleic acid sequence set forth in SEQ ID NO: 16. According to some embodiments, the endogenous gene encoding the polypeptide having Acyl-homoserine-lactone (AHL) synthase activity comprises a nucleic acid sequence having at least 80%, such as at least 85%, sequence identity with the nucleic acid sequence set forth in SEQ ID NO: 16. According to some embodiments, the endogenous gene encoding the polypeptide having Acyl-homoserine-lactone (AHL) synthase activity comprises a nucleic acid sequence having at least 90%, such as at least 95%, sequence identity with the nucleic acid sequence set forth in SEQ ID NO: 16.

According to some embodiments, the endogenous polypeptide having Acyl-homoserine-lactone (AHL) synthase activity comprises an amino acid sequence having at least 70%, such as at least 75%, sequence identity with the amino acid sequence set forth in SEQ ID NO: 17. According to some embodiments, the endogenous polypeptide having Acyl-homoserine-lactone (AHL) synthase activity comprises an amino acid sequence having at least 80%, such as at least 85%, sequence identity with the amino acid sequence set forth in SEQ ID NO: 17. According to some embodiments, the endogenous polypeptide having Acyl-homoserine-lactone (AHL) synthase activity comprises an amino acid sequence having at least 90%, such as at least 95%, sequence identity with the amino acid sequence set forth in SEQ ID NO: 17.

According to some embodiments, the endogenous gene encoding the polypeptide having Acyl-homoserine-lactone (AHL) synthase activity comprises a nucleic acid sequence having at least 70%, such as at least 75%, sequence identity with the nucleic acid sequence set forth in SEQ ID NO: 18. According to some embodiments, the endogenous gene encoding the polypeptide having Acyl-homoserine-lactone (AHL) synthase activity comprises a nucleic acid sequence having at least 80%, such as at least 85%, sequence identity with the nucleic acid sequence set forth in SEQ ID NO: 18. According to some embodiments, the endogenous gene encoding the polypeptide having Acyl-homoserine-lactone (AHL) synthase activity comprises a nucleic acid sequence having at least 90%, such as at least 95%, sequence identity with the nucleic acid sequence set forth in SEQ ID NO: 18.

According to some embodiments, the endogenous polypeptide having Acyl-homoserine-lactone (AHL) synthase activity comprises an amino acid sequence having at least 70%, such as at least 75%, sequence identity with the amino acid sequence set forth in SEQ ID NO: 19. According to some embodiments, the endogenous polypeptide having Acyl-homoserine-lactone (AHL) synthase activity comprises an amino acid sequence having at least 80%, such as at least 85%, sequence identity with the amino acid sequence set forth in SEQ ID NO: 19. According to some embodiments, the endogenous polypeptide having Acyl-homoserine-lactone (AHL) synthase activity comprises an amino acid sequence having at least 90%, such as at least 95%, sequence identity with the amino acid sequence set forth in SEQ ID NO: 19.

According to some embodiments, the endogenous gene encoding the polypeptide having Acyl-homoserine-lactone (AHL) synthase activity comprises a nucleic acid sequence having at least 70%, such as at least 75%, sequence identity with the nucleic acid sequence set forth in SEQ ID NO: 20. According to some embodiments, the endogenous gene encoding the polypeptide having Acyl-homoserine-lactone (AHL) synthase activity comprises a nucleic acid sequence having at least 80%, such as at least 85%, sequence identity with the nucleic acid sequence set forth in SEQ ID NO: 20. According to some embodiments, the endogenous gene encoding the polypeptide having Acyl-homoserine-lactone (AHL) synthase activity comprises a nucleic acid sequence having at least 90%, such as at least 95%, sequence identity with the nucleic acid sequence set forth in SEQ ID NO: 20.

According to some embodiments, the endogenous polypeptide having Acyl-homoserine-lactone (AHL) synthase activity comprises an amino acid sequence having at least 70%, such as at least 75%, sequence identity with the amino acid sequence set forth in SEQ ID NO: 21. According to some embodiments, the endogenous polypeptide having Acyl-homoserine-lactone (AHL) synthase activity comprises an amino acid sequence having at least 80%, such as at least 85%, sequence identity with the amino acid sequence set forth in SEQ ID NO: 21. According to some embodiments, the endogenous polypeptide having Acyl-homoserine-lactone (AHL) synthase activity comprises an amino acid sequence having at least 90%, such as at least 95%, at least 98% or at least 99%, sequence identity with the amino acid sequence set forth in SEQ ID NO: 21.

According to some embodiments, the endogenous gene encoding the polypeptide having Acyl-homoserine-lactone (AHL) synthase activity comprises a nucleic acid sequence having at least 70%, such as at least 75%, sequence identity with the nucleic acid sequence set forth in SEQ ID NO: 22. According to some embodiments, the endogenous gene encoding the polypeptide having Acyl-homoserine-lactone (AHL) synthase activity comprises a nucleic acid sequence having at least 80%, such as at least 85%, sequence identity with the nucleic acid sequence set forth in SEQ ID NO: 22. According to some embodiments, the endogenous gene encoding the polypeptide having Acyl-homoserine-lactone (AHL) synthase activity comprises a nucleic acid sequence having at least 90%, such as at least 95%, sequence identity with the nucleic acid sequence set forth in SEQ ID NO: 22.

According to some embodiments, the endogenous polypeptide having Acyl-homoserine-lactone (AHL) synthase activity comprises an amino acid sequence having at least 70%, such as at least 75%, sequence identity with the amino acid sequence set forth in SEQ ID NO: 23. According to some embodiments, the endogenous polypeptide having Acyl-homoserine-lactone (AHL) synthase activity comprises an amino acid sequence having at least 80%, such as at least 85%, sequence identity with the amino acid sequence set forth in SEQ ID NO: 23. According to some embodiments, the endogenous polypeptide having Acyl-homoserine-lactone (AHL) synthase activity comprises an amino acid sequence having at least 90%, such as at least 95%, sequence identity with the amino acid sequence set forth in SEQ ID NO: 23.

According to some embodiments, the endogenous gene encoding the polypeptide having Acyl-homoserine-lactone (AHL) synthase activity comprises a nucleic acid sequence having at least 70%, such as at least 75%, sequence identity with the nucleic acid sequence set forth in SEQ ID NO: 24. According to some embodiments, the endogenous gene encoding the polypeptide having Acyl-homoserine-lactone (AHL) synthase activity comprises a nucleic acid sequence having at least 80%, such as at least 85%, sequence identity with the nucleic acid sequence set forth in SEQ ID NO: 24. According to some embodiments, the endogenous gene encoding the polypeptide having Acyl-homoserine-lactone (AHL) synthase activity comprises a nucleic acid sequence having at least 90%, such as at least 95%, sequence identity with the nucleic acid sequence set forth in SEQ ID NO: 24.

According to some embodiments, the endogenous polypeptide having Acyl-homoserine-lactone (AHL) synthase activity comprises an amino acid sequence having at least 70%, such as at least 75%, sequence identity with the amino acid sequence set forth in SEQ ID NO: 25. According to some embodiments, the endogenous polypeptide having Acyl-homoserine-lactone (AHL) synthase activity comprises an amino acid sequence having at least 80%, such as at least 85%, sequence identity with the amino acid sequence set forth in SEQ ID NO: 25. According to some embodiments, the endogenous polypeptide having Acyl-homoserine-lactone (AHL) synthase activity comprises an amino acid sequence having at least 90%, such as at least 95%, sequence identity with the amino acid sequence set forth in SEQ ID NO: 25.

According to some embodiments, the endogenous gene encoding the polypeptide having Acyl-homoserine-lactone (AHL) synthase activity comprises a nucleic acid sequence having at least 70%, such as at least 75%, sequence identity with the nucleic acid sequence set forth in SEQ ID NO: 26. According to some embodiments, the endogenous gene encoding the polypeptide having Acyl-homoserine-lactone (AHL) synthase activity comprises a nucleic acid sequence having at least 80%, such as at least 85%, sequence identity with the nucleic acid sequence set forth in SEQ ID NO: 26. According to some embodiments, the endogenous gene encoding the polypeptide having Acyl-homoserine-lactone (AHL) synthase activity comprises a nucleic acid sequence having at least 90%, such as at least 95%, sequence identity with the nucleic acid sequence set forth in SEQ ID NO: 26.

According to some embodiments, the bacterium of the invention may be modified to have a decreased expression of an endogenous polypeptide having Acyl-homoserine-lactone (AHL) synthase activity compared to an otherwise identical bacterium that does not carry said modification.

According to some embodiments, the bacterium of the invention may be modified to have a decreased expression of an endogenous polypeptide having Acyl-homoserine-lactone (AHL) synthase activity compared to an otherwise identical bacterium that does not carry said modification. The expression level of the endogenous polypeptide having Acyl-homoserine-lactone (AHL) synthase activity may, for example, be decreased by at least 50%, such as by at least 60%, at least 65%, at least 70%, at least 75%, at least 80%, at least 85%, at least 90%, at least 95% or at least 100% compared to the otherwise identical bacterium.

According to some embodiments, the bacterium of the invention may be modified to have a decreased expression level of the endogenous gene encoding said endogenous polypeptide having Acyl-homoserine-lactone (AHL) synthase activity compared to an otherwise identical bacterium that does not carry said modification. The expression level of the endogenous gene may, for example, be decreased by at least 50%, such as by at least 60%, at least 65%, at least 70%, at least 75%, at least 80%, at least 85%, at least 90%, at least 95% or at least 100% compared to the otherwise identical bacterium.

According to some embodiments, the endogenous gene encoding said polypeptide having Acyl-homoserine-lactone (AHL) synthase activity has been inactivated, such as by deletion of part of or the entire gene sequence.

According to some embodiments, the endogenous gene encoding said polypeptide having Acyl-homoserine-lactone (AHL) synthase activity has been inactivated by introducing or expressing in the microorganism a rare-cutting endonuclease able to selectively inactivate by DNA cleavage, preferably by a double-strand break, the endogenous gene encoding said enzyme. A rare-cutting endonuclease to be used in accordance with the present invention to inactivate the endogenous gene may, for instance, be a transcription activator-like effector (TALE) nuclease, meganuclease, zinc-finger nuclease (ZFN), or RNA-guided endonuclease.

One way to inactivate the endogenous gene encoding said polypeptide having Acyl-homoserine-lactone (AHL) synthase activity is to use the CRISPRi system. The CRISPRi system was developed as a tool for targeted repression of gene expression or for blocking targeted locations on the genome. The CRISPRi system consists of the catalytically inactive, "dead" Cas9 protein (dCas9) and a guide RNA that defines the binding site for the dCas9 to DNA.

Thus, according to some embodiments, the endogenous gene encoding said polypeptide having Acyl-homoserine-lactone (AHL) synthase activity is inactivated by introducing or expressing in the bacterium an RNA-guided endonuclease, such as a catalytically inactive Cas9 protein, and a single guide RNA (sgRNA) specifically hybridizing (e.g. binding) under cellular conditions with the genomic DNA encoding a said polypeptide.

According to some embodiments, the expression of said endogenous polypeptide having Acyl-homoserine-lactone (AHL) synthase activity is decreased by way of inhibition.

Inhibition of the expression of the said endogenous polypeptide may be achieved by any suitable means known in the art. For example, the expression may be inhibited by gene silencing techniques involving the use of inhibitory nucleic acid molecules, such as antisense oligonucleotides, ribozymes, or interfering RNA (RNAi) molecules, such as microRNA (miRNA), small interfering RNA (siRNA), or short hairpin RNA (shRNA).

According to some embodiments, the expression of said endogenous polypeptide having Acyl-homoserine-lactone (AHL) synthase activity is decreased (e.g., inhibited) by transcriptional and/or translational repression of the endogenous gene encoding said polypeptide.

According to some embodiments, the expression of endogenous polypeptide having Acyl-homoserine-lactone (AHL) synthase activity is inhibited by introducing or expressing in the bacterium an inhibitory nucleic acid molecule. For example, the inhibitory nucleic acid molecule may be introduced by way of an exogenous nucleic acid molecule comprising a nucleotide sequence encoding said inhibitory nucleic acid molecule operably linked to a promoter, such as an inducible promoter, that is functional in the bacterium to cause the production of said inhibitory nucleic acid molecule. Suitably, the inhibitory nucleic acid molecule is one that specifically hybridizes (e.g. binds) under cellular conditions with cellular mRNA and/or genomic DNA encoding the endogenous polypetide. Depending on the target, transcription of the encoding genomic DNA and/or translation of the encoding mRNA is/are inhibited.

According to some embodiments, the inhibitory nucleic acid molecule is an antisense oligonucleotide, ribozyme, or interfering RNA (RNAi) molecule. Preferably, such nucleic acid molecule comprises at least 10 consecutive nucleotides of the complement of the cellular mRNA and/or genomic DNA encoding the polypeptide or enzyme of interest (e.g., the cellular mRNA and/or genomic DNA encoding the polypeptide.

According to some embodiments, the inhibitory nucleic acid is an antisense oligonucleotide. Such antisense oligonucleotide is a nucleic acid molecule (either DNA or RNA), which specifically hybridizes (e.g. binds) under cellular conditions with the cellular mRNA and/or genomic DNA encoding the polypeptide.

According to some embodiments, the inhibitory nucleic acid molecule is a ribozyme, such as a hammerhead ribozyme. A ribozyme molecule is designed to catalytically cleave the mRNA transcript to prevent translation of the polypeptide.

According to some embodiments, the inhibitory nucleic acid molecule is an interfering RNA (RNAi) molecule. RNA interference is a biological process in which RNA molecules inhibit expression, typically destroying specific mRNA. Exemplary types of RNAi molecules include microRNA (miRNA), small interfering RNA (siRNA), and short hairpin RNA (shRNA). According to some embodiments, the RNAi molecule is a miRNA. According to some embodiments, the RNAi molecule is a siRNA. According to some embodiments, the RNAi molecule is an shRNA.

According to some embodiments, the bacterium of the invention has been modified to have a decreased activity of an endogenous polypeptide having Acyl-homoserine-lactone (AHL) synthase activity compared to an otherwise identical bacterium that does not carry said modification.

A decrease of the activity of the polypeptide having Acyl-homoserine-lactone (AHL) synthase activity may be achieved by any suitable means known in the art. For example, the activity may be decreased by introducing one or more mutations in the active site of the polypeptide resulting in the reduction or loss of activity. Thus, according to some embodiments, the activity of the endogenous polypeptide having Acyl-homoserine-lactone (AHL) synthase activity is decreased by at least one active-site mutation resulting in the reduction or loss of activity. At least one active-site mutation may, for example, be at least one non-conservative amino acid substitution.

Generally, a bacterium as referred to herein may be any suitable bacterium, provided it contains within its genome a gene encoding a polypeptide having polyphosphate kinase activity. The bacterium may be Gram-positive or Gram-negative. Preferably, the bacterium is a Gram-negative bacterium. Non-limiting examples for Gram-negative bacteria include species from the genera *Methylobacillus, Methylobacterium, Methylorubrum, Escherichia, Erwinia, Klebsiella and Citrobacter. Non-limiting examples of Gram-positive bacteria include species from the genera Bacillus, Lactococcus, Lactobacillus, Geobacillus, Pediococcus, Moorella, Clostridium, Corynebacterium, Streptomyces, Streptococcus, and Cellulomonas.*

According to some embodiments, the bacterium of the present invention is a methylotrophic bacterium, preferably a mesophilic, methylotrophic bacterium.

According to some embodiments, the bacterium of the present invention belongs to the family *Methylophilaceae* or *Methylobacteriaceae.*

According to some embodiments, the bacterium of the present invention belongs to the family *Methylophilaceae.*

According to some embodiments, the bacterium of the present invention belongs to the family *Methylobacteriaceae.*

According to some embodiments, the bacterium of the present invention belongs to the genus *Methylobacillus, Methylobacterium or Methylorubrum.*

According to some embodiments, the bacterium of the present invention belongs to the genus *Methylobacillus* or *Methylobacterium.*

According to some embodiments, the bacterium of the present invention belongs to the genus *Methylobacterium or Methylorubrum.*

According to some embodiments, the bacterium of the present invention is selected from *Methylobacillus flagellatus, Methylobacillus glycogenes, Methylobacillus pratensis, Methylobacillus rhizosphaerae, Methylobacillus gramineus, Methylobacillus arboreus, Methylobacillus caricics,* *Methylobacillus methilovorans, Methylobacillus sp, Methylobacterium extorquens, Methylobacterium organophilum and Methylorubrum extorquens.*

According to some embodiments, the bacterium of the present invention belongs to the genus *Methylobacillus.*

According to some embodiments, the bacterium of the present invention is selected from *Methylobacillus flagellatus, Methylobacillus glycogenes, Methylobacillus pratensis, Methylobacillus rhizosphaerae, Methylobacillus gramineus, Methylobacillus arboreus, Methylobacillus caricics, Methylobacillus methilovorans,* and *Methylobacillus sp.*

According to some embodiments, the bacterium of the present invention is *Methylobacillus flagellutes.*

According to some embodiments, the bacterium of the present invention is *Methylobacillus glycogenes.*

According to some embodiments, the bacterium of the present invention is *Methylobacillus rhizosphaerae.*

According to some embodiments, the bacterium of the present invention is of the family *Enterobacteriaceae.*

According to some embodiments, the bacterium of the present invention belongs to the genus *Escherichia.*

According to some embodiments, the bacterium of the present invention is *Escherichia coli.*

The present inventors have further studied general EPS production in *Methylobacilli.* Based on bioinformatic analysis of the genome of *M. flagellatus* and *M. glycogenes,* the present inventors identified two putative EPS-producing gene clusters. Disruption of both EPS clusters resulted in complete abolishment of EPS production.

An unexpected and surprising observation in the modified *M. flagellatus* and M. *glycogenes* strains was their behavior in growth cultures. When abolishing the expression of one or more endogenous polypeptides involved in the production of exopolysaccharides (EPS) in the *Methylobacillus* bacteria, the culture showed a number of surprising and unexpected properties beneficial to the development of methanol-based bioprocesses. Unexpectedly, the excessive culture foaming in both shake flasks and bioreactors was reduced. Moreover, while cell clumping was observed for unmodified parent strains, this was reduced or even absent in the culture of modified Methylobacilli. Centrifugation and filtration of the biomass was also improved when EPS production was eliminated. These combined characteristics significantly improve the bioprocess, strain handling, and downstream processing, making modified *Methylobacillus* bacteria suitable for large-scale methanol fermentations. Removal of EPS synthesis unexpectedly also improved the methanol tolerance of *M. flagellatus* and other *Methylobacillus* species.

Thus, according to some embodiments, a bacterium of the present invention, which is of the genus *Methylobacillus,* may be further modified to have a decreased production of exopolysaccharides (EPS) compared to an otherwise identical bacterium that does not carry said modification. More specifically, such genetically engineered bacterium of the genus *Methylobacillus* may be modified to have a decreased expression and/or activity of at least one endogenous polypeptide involved in the production of exopolysaccharides (EPS) in said bacterium compared to an otherwise identical bacterium that does not carry said modification.

According to some embodiments, the bacterium of the invention has been modified to have a decreased expression of at least one, such as at least two, endogenous polypeptide involved in the production of exopolysaccharides (EPS) in said bacterium compared to an otherwise identical bacterium that does not carry said modification.

According to some embodiments, the bacterium of the invention has been modified to have a decreased expression of at least three, such as at least four, endogenous polypeptides involved in the production of exopolysaccharides (EPS) in said bacterium compared to an otherwise identical bacterium that does not carry said modification.

According to some embodiments, the bacterium of the invention has been modified to have a decreased expression of at least five, such as at least six, endogenous polypeptides involved in the production of exopolysaccharides (EPS) in said bacterium compared to an otherwise identical bacterium that does not carry said modification.

According to some embodiments, the bacterium of the invention has been modified to have a decreased expression of at least seven, such as at least eight, endogenous polypeptides involved in the production of exopolysaccharides (EPS) in said bacterium compared to an otherwise identical bacterium that does not carry said modification.

According to some embodiments, the bacterium of the invention has been modified to have a decreased expression of at least nine, such as at least ten, endogenous polypeptides involved in the production of exopolysaccharides (EPS) in said bacterium compared to an otherwise identical bacterium that does not carry said modification.

According to some embodiments, the bacterium of the invention has been modified to have a decreased expression of at least eleven, such as at least twelve, endogenous polypeptides involved in the production of exopolysaccharides (EPS) in said bacterium compared to an otherwise identical bacterium that does not carry said modification.

According to some embodiments, the bacterium of the invention has been modified to have a decreased expression of at least thirteen, such as at least fourteen, endogenous polypeptides involved in the production of exopolysaccharides (EPS) in said bacterium compared to an otherwise identical bacterium that does not carry said modification.

According to some embodiments, the bacterium of the invention has been modified to have a decreased expression of at least fifteen, such as at least sixteen, endogenous polypeptides involved in the production of exopolysaccharides (EPS) in said bacterium compared to an otherwise identical bacterium that does not carry said modification.

According to some embodiments, the bacterium of the invention has been modified to have a decreased expression of all endogenous polypeptides involved in the production of exopolysaccharides (EPS) in said bacterium compared to an otherwise identical bacterium that does not carry said modification.

According to some embodiments, the at least one polypeptide involved in the production of exopolysaccharides (EPS) in said bacterium is selected from the group consisting of a) polypeptides comprising an amino acid sequence set forth in any one of SEQ ID NO: 27 to 64 and b) polypeptides comprising an amino acid sequence having at least 70%%, such as at least 75%, at least 80%, at least 85%, at least 90% or at least 95%, sequence identity with any one of SEQ ID NO: 27 to 64 and having the same functional property as the reference polypeptide.

According to some embodiments, the at least one polypeptide involved in the production of exopolysaccharides (EPS) in said bacterium is selected from the group consisting of a) polypeptides comprising an amino acid sequence set forth in any one of SEQ ID NO: 27 to 52 and b) polypeptides comprising an amino acid sequence having at least 70%%, such as at least 75%, at least 80%, at least 85%, at least 90% or at least 95%, sequence identity with any one of SEQ ID NO: 27 to 52 and having the same functional property as the reference polypeptide.

According to some embodiments, the at least one polypeptide involved in the production of exopolysaccharides (EPS) in said bacterium is selected from the group consisting of a) polypeptides comprising an amino acid sequence set forth in any one of SEQ ID NO: 53 to 64 and b) polypeptides comprising an amino acid sequence having at least 70%%, such as at least 75%, at least 80%, at least 85%, at least 90% or at least 95%, sequence identity with any one of SEQ ID NO: 53 to 64 and having the same functional property as the reference polypeptide.

According to some embodiments, the at least one polypeptide involved in the production of exopolysaccharides (EPS) in said bacterium is selected from the group consisting of a) polypeptides comprising an amino acid sequence set forth in any one of SEQ ID NO: 65 to 112 and b) polypeptides comprising an amino acid sequence having at least 70%, such as at least 75%, at least 80%, at least 85%, at least 90% or at least 95%, sequence identity with any one of SEQ ID NO: 65 to 112 and having the same functional property as the reference polypeptide.

According to some embodiments, the at least one polypeptide involved in the production of exopolysaccharides (EPS) in said bacterium is selected from the group consisting of a) polypeptides comprising an amino acid sequence set forth in any one of SEQ ID NO: 65 to 86 and b) polypeptides comprising an amino acid sequence having at least 70%, such as at least 75%, at least 80%, at least 85%, at least 90% or at least 95%, sequence identity with any one of SEQ ID NO: 65 to 86 and having the same functional property as the reference polypeptide.

According to some embodiments, the at least one enzyme involved in the production of exopolysaccharides (EPS) in said bacterium is selected from the group consisting of a) polypeptides comprising an amino acid sequence set forth in any one of SEQ ID NO: 87 to 112 and b) polypeptides comprising an amino acid sequence having at least 70%%, such as at least 75%, at least 80%, at least 85%, at least 90% or at least 95%, sequence identity with any one of SEQ ID NO: 87 to 112 and having the same functional property as the reference polypeptide.

According to some embodiments, the at least one polypeptide involved in the production of exopolysaccharides (EPS) in said bacterium is selected from the group consisting a polypeptide having peptidyl-prolyl cis-trans isomerase activity, a polypeptide capable of acting as a polysaccharide exporter; a polypeptide acting as a chain length determinant protein; and a polypeptide having protein-tyrosine kinase activity.

According to some embodiments, the polypeptide having peptidyl-prolyl cis-trans isomerase activity comprises an amino acid sequence having at least 70%, such as at least 85%, sequence identity to SEQ ID NO: 32.

According to some embodiments, the polypeptide capable of acting as a polysaccharide exporter comprises an amino acid sequence having at least 70%, such as at least 85%, sequence identity to SEQ ID NO: 33.

According to some embodiments, the polypeptide acting as a chain length determinant protein comprises an amino acid sequence having at least 70%, such as at least 85%, sequence identity to SEQ ID NO: 34.

According to some embodiments, the polypeptide having protein-tyrosine kinase activity comprises an amino acid sequence having at least 70%, such as at least 85%, sequence identity to SEQ ID NO: 35.

According to some embodiments, the polypeptide having peptidyl-prolyl cis-trans isomerase activity comprises an amino acid sequence having at least 70%, such as at least 85%, sequence identity to SEQ ID NO: 68.

According to some embodiments, the polypeptide capable of acting as a polysaccharide exporter comprises an amino acid sequence having at least 70%, such as at least 85%, sequence identity to SEQ ID NO: 69.

According to some embodiments, the polypeptide acting as a chain length determinant protein comprises an amino acid sequence having at least 70%, such as at least 85%, sequence identity to SEQ ID NO: 70.

According to some embodiments, the polypeptide having protein-tyrosine kinase activity comprises an amino acid sequence having at least 70%, such as at least 85%, sequence identity to SEQ ID NO: 71.

The expression level of the endogenous polypeptide(s) may, for example, be decreased by at least 50%, such as by at least 60%, at least 65%, at least 70%, at least 75%, at least 80%, at least 85%, at least 90%, at least 95% or at least 100% compared to the otherwise identical bacterium.

A decrease of the expression of the endogenous polypeptide(s) may be achieved by any suitable means known in the art. For example, the expression may be decrease by inactivating the endogenous gene(s) encoding said polypeptide(s) involved in the production of exopolysaccharides (EPS) in the bacterium, such as by deletion of part of or the entire gene sequence.

According to some embodiments, the expression of the at least one polypeptide involved in the production of exopolysaccharides (EPS) in said bacterium is abolished compared to the otherwise identical bacterium.

According to some embodiments, the endogenous gene/s encoding said polypeptide/s involved in the production of exopolysaccharides (EPS) in the bacterium has/have been inactivated, such as by deletion of part of or the entire gene sequence.

According to some embodiments, the endogenous gene/s encoding said polypeptide/s involved in the production of exopolysaccharides (EPS) in said bacterium has/have been inactivated by introducing or expressing in the bacterium a rare-cutting endonuclease able to selectively inactivating by DNA cleavage the endogenous gene encoding said enzyme. A rare-cutting endonuclease to be used in accordance of the present invention to inactivate the endogenous gene may, for instance, be a transcription activator-like effector (TALE) nuclease, meganuclease, zing-finger nuclease (ZFN), or RNA-guided endonuclease.

One way to inactivate an endogenous gene encoding a polypeptide involved in the production of exopolysaccharides (EPS) in the bacterium is to use the CRISPRi system. The CRISPRi system was developed as a tool for targeted repression of gene expression or for blocking targeted locations on the genome. The CRISPRi system consists of the catalytically inactive, "dead" Cas9 protein (dCas9) and a guide RNA that defines the binding site for the dCas9 to DNA.

According to some embodiments, the expression of an endogenous polypeptide involved in the production of exopolysaccharides (EPS) in the bacterium is decreased by way of inhibition.

Inhibition of the expression of said polypeptide may be achieved by any suitable means known in the art. For example, the expression may be inhibited by gene silencing techniques involving the use of inhibitory nucleic acid molecules, such as antisense oligonucleotides, ribozymes or interfering RNA (RNAi) molecules, such as microRNA (miRNA), small interfering RNA (siRNA) or short hairpin RNA (shRNA).

According to some embodiments, the expression of an endogenous polypeptide involved in the production of exopolysaccharides (EPS) in the bacterium is decreased (e.g., inhibited) by transcriptional and/or translational repression of the endogenous gene encoding said polypeptide.

According to some embodiments, the expression of an endogenous polypeptide involved in the production of exopolysaccharides (EPS) in the bacterium is inhibited by introducing or expressing in the bacterium an inhibitory nucleic acid molecule. For example, the inhibitory nucleic acid molecule may be introduced by way of an exogenous nucleic acid molecule comprising a nucleotide sequence encoding said inhibitory nucleic acid molecule operably linked to a promoter, such as an inducible promoter, that is functional in the bacterium to cause the production of said inhibitory nucleic acid molecule. Suitably, the inhibitory nucleic acid molecule is one that specifically hybridizes (e.g. binds) under cellular conditions with cellular mRNA and/or genomic DNA encoding the endogenous polypeptide in question. Depending on the target, transcription of the encoding genomic DNA and/or translation of the encoding mRNA is/are inhibited.

According to some embodiments, the inhibitory nucleic acid molecule is an antisense oligonucleotide, ribozyme or interfering RNA (RNAi) molecule. Preferably, such nucleic acid molecule comprises at least 10 consecutive nucleotides of the complement of the cellular mRNA and/or genomic DNA encoding the polypeptide in question.

According to some embodiments, the inhibitory nucleic acid is an antisense oligonucleotide. Such antisense oligonucleotide is a nucleic acid molecule (either DNA or RNA) which specifically hybridizes (e.g. binds) under cellular conditions with the cellular mRNA and/or genomic DNA encoding the enzyme in question.

According to some embodiments, the inhibitory nucleic acid molecule is a ribozyme, such as a hammerhead ribozyme. A ribozyme molecule is designed to catalytically cleave the mRNA transcript to prevent translation of the polypeptide in question.

According to some embodiments, the inhibitory nucleic acid molecule is an interfering RNA (RNAi) molecule. RNA interference is a biological process in which RNA molecules inhibit expression, typically causing the destruction of specific mRNA. Exemplary types of RNAi molecules include microRNA (miRNA), small interfering RNA (siRNA) and short hairpin RNA (shRNA). According to some embodiments, the RNAi molecule is a miRNA. According to some embodiments, the RNAi molecule is a siRNA. According to some embodiments, the RNAi molecule is a shRNA.

According to some embodiments, bacterium of the invention has been modified to have a decreased function (e.g. activity) of at least one polypeptide involved in the production of exopolysaccharides (EPS) in the bacterium compared to an otherwise identical microorganism that does not carry said modification.

A decrease of the function (e.g. activity) of the at least one endogenous polypeptide may be achieved by any suitable means known in the art. For example, if the polypeptide is an enzyme the activity may be decrease by introducing one or more mutations in the active site of the enzyme resulting in the reduction or loss of activity. Thus, according to some embodiments, the activity of the at least one endogenous enzyme involved in the production of exopolysaccharides (EPS) in the bacterium is decreased by at least one active-site mutation resulting in the reduction or loss of activity. The at least one active-site mutation may, for example, be at least one non-conservative amino acid substitution.

As noted above, the present inventors have identified two putative EPS gene clusters in the genome of *Methylobacilli.* The endogenous polypeptide or polypeptides involved in the production of exopolysaccharides (EPS) is/are thus encoded by a gene or genes included in a first EPS gene cluster and/or second EPS gene cluster.

According to some embodiments, the endogenous polypeptide or polypeptides involved in the production of exopolysaccharides (EPS) in said bacterium is/are encoded by a gene or genes included in a first EPS gene cluster and/or second EPS gene cluster.

According to some embodiments, the first EPS gene cluster includes genes defined by or orthologous to the open reading frames (ORFs) found in SEQ ID NO: 200 or 203.

According to some embodiments, the first EPS gene cluster includes the genes *epsD, epsE, epsF, epsG, epsB, epsL, epsH, epsl, epsJ* and *epsS,* or orthologs thereof.

According to some embodiments, the first EPS gene cluster includes the genes *epsD, epsE, epsF* and *epsG,* or orthologs thereof.

According to some embodiments, the first EPS gene cluster includes a nucleotide sequence which has at least 50%, such as at least 55%, sequence identity with the nucleotide sequence of SEQ ID NO: 200 or 203. According to some embodiments, the first EPS gene cluster includes a nucleotide sequence which has at least 60%, such as at least 65%, sequence identity with the nucleotide sequence of SEQ ID NO: 200 or 203. According to some embodiments, the first EPS gene cluster includes a nucleotide sequence which has at least 70%, such as at least 75%, sequence identity with the nucleotide sequence of SEQ ID NO: 200 or 203. According to some embodiments, the first EPS gene cluster includes a nucleotide sequence which has at least 80%, such as at least 85%, sequence identity with the nucleotide sequence of SEQ ID NO: 200 or 203. According to some embodiments, the first EPS gene cluster includes a nucleotide sequence which has at least 90%, such as at least 93%, sequence identity with the nucleotide sequence of SEQ ID NO: 200 or 203. According to some embodiments, the first EPS gene cluster includes a nucleotide sequence which has at least 95%, such as at least 97%, sequence identity with the nucleotide sequence of SEQ ID NO: 200 or 203.

According to some embodiments, the first EPS gene cluster includes a nucleotide sequence which has at least 50%, such as at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%at least, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95% at least 96%, at least 97%, at least 98% or at least 99%, sequence identity with the nucleotide sequence of SEQ ID NO: 200.

According to some embodiments, the first EPS gene cluster includes a nucleotide sequence which has at least 50%, such as at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%at least, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95% at least 96%, at least 97%, at least 98% or at least 99%, sequence identity with the nucleotide sequence of SEQ ID NO: 203.

According to some embodiments, the second EPS gene cluster includes genes defined by or orthologous to the open reading frames (ORFs) found in SEQ ID NO: 201 or 205.

According to some embodiments, the second EPS gene cluster includes a nucleotide sequence which has at least 50%, such as at least 55%, sequence identity with the nucleotide sequence of SEQ ID NO: 201 or 205. According to some embodiments, the second EPS gene cluster includes a nucleotide sequence which has at least 60%, such as at least 65%, sequence identity with the nucleotide sequence of SEQ ID NO: 201 or 205. According to some embodiments, the second EPS gene cluster includes a nucleotide sequence which has at least 70%, such as at least 75%, sequence identity with the nucleotide sequence of SEQ ID NO: 201 or 205. According to some embodiments, the second EPS gene cluster includes a nucleotide sequence which has at least 80%, such as at least 85%, sequence identity with the nucleotide sequence of SEQ ID NO: 201 or 205. According to some embodiments, the second EPS gene cluster includes a nucleotide sequence which has at least 90%, such as at least 85%, sequence identity with the nucleotide sequence of SEQ ID NO: 201 or 205.

According to some embodiments, the second EPS gene cluster includes a nucleotide sequence which has at least 50%, such as at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%at least, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95% at least 96%, at least 97%, at least 98% or at least 99%, sequence identity with the nucleotide sequence of 201.

According to some embodiments, the second EPS gene cluster includes a nucleotide sequence which has at least 50%, such as at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%at least, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95% at least 96%, at least 97%, at least 98% or at least 99%, sequence identity with the nucleotide sequence of 205.

According to some embodiments, at least one gene in said first EPS gene cluster is inactivated, e.g., by deletion of part of or the entire gene sequence.

According to some embodiments, the at least one gene is selected from *epsD, epsE, epsF, epsG, epsB, epsL, epsH, epsl, epsJ* and *epsS,* or ortholog thereof.

According to some embodiments, the at least one gene is selected from *epsD, epsE, epsF* and *epsG,* or ortholog thereof.

According to some embodiments, the genes *epsD, epsE, epsF* and *epsG,* or orthologs thereof, are inactivated, e.g., by deletion of part of or the entire gene sequence.

According to some embodiments, the gene *epsD* encodes a polypeptide comprising an amino acid sequence having at least 70%, such as at least 75%, sequence identity to SEQ ID NO: 32, the gene *epsE* encodes a polypeptide comprising an amino acid sequence having at least 70%, such as at least 75%, sequence identity to SEQ ID NO: 33, the gene *epsF* encodes a polypeptide comprising an amino acid sequence having at least 70%, such as at least 75%, sequence identity to SEQ ID NO: 34, and the gene *epsG* encodes a polypeptide comprising an amino acid sequence having at least 70%, such as at least 75%, sequence identity to SEQ ID NO: 35.

According to some embodiments, the gene *epsD* encodes a polypeptide comprising an amino acid sequence having at least 80%, such as at least 85%, sequence identity to SEQ ID NO: 32, the gene *epsE* encodes a polypeptide comprising an amino acid sequence having at least 80%, such as at least 85%, sequence identity to SEQ ID NO: 33, the gene *epsF* encodes a polypeptide comprising an amino acid sequence having at least 80%, such as at least 85, sequence identity to SEQ ID NO: 34, and the gene *epsG* encodes a polypeptide comprising an amino acid sequence having at least 80%, such as at least 85%, sequence identity to SEQ ID NO: 35.

According to some embodiments, the gene *epsD* encodes a polypeptide comprising an amino acid sequence having at least 90%, such as at least 95%, sequence identity to SEQ ID NO: 32, the gene *epsE* encodes a polypeptide comprising an amino acid sequence having at least 90%, such as at least 95%, sequence identity to SEQ ID NO: 33, the gene *epsF* encodes a polypeptide comprising an amino acid sequence having at least 90%, such as at least 95, sequence identity to SEQ ID NO: 34, and the gene *epsG* encodes a polypeptide comprising an amino acid sequence having at least 90%, such as at least 95%, sequence identity to SEQ ID NO: 35.

According to some embodiments, the *epsD* gene comprises a nucleotide sequence having at least 70%, such as at least 75%, sequence identity with SEQ ID NO: 118, the gene *epsE* comprises a nucleotide sequence having at least 70%, such as at least 75%, sequence identity with SEQ ID NO: 119, the gene *epsF* comprises an nucleotide sequence having at least 70%, such as at least 75% sequence identity with SEQ ID NO: 120, and the gene *epsG* comprises an nucleotide sequence having at least 70%, such as at least 75%, sequence identity with SEQ ID NO: 121.

According to some embodiments, the *epsD* gene comprises a nucleotide sequence having at least 80%, such as at least 85%, sequence identity with SEQ ID NO: 118, the gene *epsE* comprises a nucleotide sequence having at least 80%, such as at least 85%, sequence identity with SEQ ID NO: 119, the gene *epsF* comprises an nucleotide sequence having at least 80%, such as at least 85% sequence identity with SEQ ID NO: 120, and the gene *epsG* comprises an nucleotide sequence having at least 80%, such as at least 85%, sequence identity with SEQ ID NO: 121.

According to some embodiments, the *epsD* gene comprises a nucleotide sequence having at least 90%, such as at least 95%, sequence identity with SEQ ID NO: 118, the gene *epsE* comprises a nucleotide sequence having at least 90%, such as at least 95%, sequence identity with SEQ ID NO: 119, the gene *epsF* comprises an nucleotide sequence having at least 90%, such as at least 95% sequence identity with SEQ ID NO: 120, and the gene *epsG* comprises an nucleotide sequence having at least 90%, such as at least 95% sequence identity with SEQ ID NO: 121.

According to some embodiments, the gene *epsD* encodes a polypeptide comprising an amino acid sequence having at least 70%, such as at least 75%, sequence identity to SEQ ID NO: 68, the gene *epsE* encodes a polypeptide comprising an amino acid sequence having at least 70%, such as at least 75%, sequence identity to SEQ ID NO: 69, the gene epsFencodes a polypeptide comprising an amino acid sequence having at least 70%, such as at least 75%, sequence identity to SEQ ID NO: 70, and the gene *epsG* encodes a polypeptide comprising an amino acid sequence having at least 70%, such as at least 75%, sequence identity to SEQ ID NO: 71.

According to some embodiments, the gene *epsD* encodes a polypeptide comprising an amino acid sequence having at least 80%, such as at least 85%, sequence identity to SEQ ID NO: 68, the gene *epsE* encodes a polypeptide comprising an amino acid sequence having at least 80%, such as at least 85%, sequence identity to SEQ ID NO: 69, the gene epsFencodes a polypeptide comprising an amino acid sequence having at least 80%, such as at least 85%, sequence identity to SEQ ID NO: 70, and the gene *epsG* encodes a polypeptide comprising an amino acid sequence having at least 80%, such as at least 85%, sequence identity to SEQ ID NO: 71.

According to some embodiments, the gene *epsD* encodes a polypeptide comprising an amino acid sequence having at least 90%, such as at least 95%, sequence identity to SEQ ID NO: 68, the gene *epsE* encodes a polypeptide comprising an amino acid sequence having at least 90%, such as at least 95%, sequence identity to SEQ ID NO: 69, the gene epsFencodes a polypeptide comprising an amino acid sequence having at least 90%, such as at least 95%, sequence identity to SEQ ID NO: 70, and the gene *epsG* encodes a polypeptide comprising an amino acid sequence having at least 90%, such as at least 95%, sequence identity to SEQ ID NO: 71.

According to some embodiments, the gene *epsD* comprises a nucleotide sequence having at least 70%, such as at least 75%, sequence identity with SEQ ID NO: 154, the gene *epsE* comprises a nucleotide sequence having at least 70%, such as at least 75%, sequence identity with SEQ ID NO: 155, the gene *epsF* comprises an nucleotide sequence having at least 70%, such as at least 75%, sequence identity with SEQ ID NO: 156, and the gene *epsG* comprises an nucleotide sequence having at least 70%, such as at least 75%, sequence identity with SEQ ID NO: 157.

According to some embodiments, the gene *epsD* comprises a nucleotide sequence having at least 80%, such as at least 85%, sequence identity with SEQ ID NO: 154, the gene *epsE* comprises a nucleotide sequence having at least 80%, such as at least 85%, sequence identity with SEQ ID NO: 155, the gene *epsF* comprises an nucleotide sequence having at least 80%, such as at least 85%, sequence identity with SEQ ID NO: 156, and the gene *epsG* comprises an nucleotide sequence having at least 80%, such as at least 85%, sequence identity with SEQ ID NO: 157.

According to some embodiments, the gene *epsD* comprises a nucleotide sequence having at least 90%, such as at least 95%, sequence identity with SEQ ID NO: 154, the gene *epsE* comprises a nucleotide sequence having at least 90%, such as at least 95%, sequence identity with SEQ ID NO: 155, the gene *epsF* comprises an nucleotide sequence having at least 90%, such as at least 95%, sequence identity with SEQ ID NO: 156, and the gene *epsG* comprises an nucleotide sequence having at least 90%, such as at least 95%, sequence identity with SEQ ID NO: 157.

According to some embodiments, the genetically engineered bacterium of the invention has been modified to inactivate the first EPS gene cluster.

According to some embodiments, the genetically engineered bacterium of the invention has been (further) modified to inactivate the second EPS gene cluster.

According to some embodiments, the genetically engineered bacterium of the invention has been modified to inactivate the first EPS gene cluster and second EPS gene cluster.

According to some embodiments, the first EPS gene cluster is inactivated by deletion of part of or the entire sequence of said cluster.

According to some embodiments, the first EPS gene cluster is inactivated by deletion of the entire coding sequence of said cluster.

According to some embodiments, the first EPS gene cluster is inactivated by deletion of the entire sequence of said cluster.

According to some embodiments, the first EPS gene cluster is inactivated by modification of (e.g., by introducing at least one mutation in) the promoter and/or ribosome binding site region resulting in the lack of gene expression.

According to some embodiments, the second EPS gene cluster is inactivated by deletion of part of or the entire sequence of said cluster.

According to some embodiments, the second EPS gene cluster is inactivated by deletion of the entire coding sequence of said cluster.

According to some embodiments, the second EPS gene cluster is inactivated by deletion of the entire sequence of said cluster.

According to some embodiments, the second EPS gene cluster is inactivated by modification of (e.g., by introducing at least one mutation in) the promoter and/or ribosome binding site region resulting in the lack of gene expression.

According to some embodiments, the genetically engineered bacterium of the invention has been modified to delete a nucleotide sequence having at least 70%, such as at least 75%, sequence identity with SEQ ID NO: 207 or 209. According to some embodiments, the genetically engineered bacterium of the invention has been modified to delete a nucleotide sequence having at least 80%, such as at least 85%, sequence identity with SEQ ID NO: 207 or 209. According to some embodiments, the genetically engineered bacterium of the invention has been modified to delete a nucleotide sequence having at least 90%, such as at least 95%, sequence identity with SEQ ID NO: 207 or 209.

According to some embodiments, the genetically engineered bacterium of the invention has been modified to delete a nucleotide sequence having at least 70%, such as at least 75%, sequence identity with SEQ ID NO: 208 or 210. According to some embodiments, the genetically engineered bacterium of the invention has been modified to delete a nucleotide sequence having at least 80%, such as at least 85%, sequence identity with SEQ ID NO: 208 or 210. According to some embodiments, the genetically engineered bacterium of the invention has been modified to delete a nucleotide sequence having at least 90%, such as at least 95%, sequence identity with SEQ ID NO: 208 or 210.

According to some embodiments, the bacterium of the invention has been modified to delete a nucleotide sequence having at least 70%, such as at least 75%, sequence identity with SEQ ID NO: 207 and to delete a nucleotide sequence having at least 70%, such as at least 75%, sequence identity with SEQ ID NO: 208. According to some embodiments, the bacterium of the invention has been modified to delete a nucleotide sequence having at least 80%, such as at least 85%, sequence identity with SEQ ID NO: 207 and to delete a nucleotide sequence having at least 80%, such as at least 85%, sequence identity with SEQ ID NO: 208. According to some embodiments, the bacterium of the invention has been modified to delete a nucleotide sequence having at least 90%, such as at least 95%, sequence identity with SEQ ID NO: 207 and to delete a nucleotide sequence having at least 90%, such as at least 95%, sequence identity with SEQ ID NO: 208.

According to some embodiments, the bacterium of the invention has been modified to delete a nucleotide sequence having at least 70%, such as at least 75%, sequence identity with SEQ ID NO: 209 and to delete a nucleotide sequence having at least 70%, such as at least 75%, sequence identity with SEQ ID NO: 210. According to some embodiments, the bacterium of the invention has been modified to delete a nucleotide sequence having at least 80%, such as at least 85%, sequence identity with SEQ ID NO: 209 and to delete a nucleotide sequence having at least 80%, such as at least 85%, sequence identity with SEQ ID NO: 210. According to some embodiments, the bacterium of the invention has been modified to delete a nucleotide sequence having at least 90%, such as at least 95%, sequence identity with SEQ ID NO: 209 and to delete a nucleotide sequence having at least 90%, such as at least 95%, sequence identity with SEQ ID NO: 210.

### Method of the invention

The present invention also provides methods for producing a biochemical compound comprising cultivating a bacterium according to the invention under suitable culture conditions. The method may further comprise collecting the biochemical compound from the culture medium.

According to some embodiments, present invention provides a method for producing GABA or a derivative thereof. Particularly, the present invention provides a method for producing GABA or a derivative thereof; said method comprises cultivating a bacterium according to the invention in a culture medium. The method may further comprise collecting GABA or the derivative thereof from the culture medium. According to some embodiments, the GABA derivative is selected from the group consisting of 2-pyrrolidone, N-methyl-2-pyrrolidone and polybutyrolactam.

The culture medium employed may be any conventional medium suitable for culturing a bacterium cell in question, and may be composed according to the principles of the prior art. The medium will usually contain all nutrients necessary for the growth and survival of the respective bacterium, such as carbon and nitrogen sources and other inorganic salts. Suitable media, e.g. minimal or complex media, are available from commercial suppliers, or may be prepared according to published receipts, e.g. the American Type Culture Collection (ATCC) Catalogue of strains. Non-limiting standard medium well known to the skilled person include Luria Bertani (LB) broth, Sabouraud Dextrose (SD) broth, MS broth, Yeast Peptone Dextrose, BMMY, GMMY, or Yeast Malt Extract (YM) broth, which are all commercially available. A non-limiting example of suitable media for culturing bacterial cells, such as E. coli cells, including minimal media and rich media such as Luria Broth (LB), M9 media, M17 media, SA media, MOPS media, Terrific Broth, YT and others.

The carbon source may be any suitable carbon substrate known in the art, and in particularly any carbon substrate commonly used in the cultivation of bacteria and/ or fermentation. Non-limiting examples of suitable fermentable carbon substrates include carbohydrates (e.g., C5 sugars such as arabinose or xylose, or C6 sugars such as glucose), glycerol, glycerine, acetate, dihydroxyacetone, one-carbon source, methanol, methane, oils, animal fats, animal oils, plant oils, fatty acids, lipids, phospholipids, glycerolipids, monoglycerides, diglycerides, triglycerides, renewable carbon sources, polypeptides (e.g., a microbial or plant protein or peptide), yeast extract, component from a yeast extract, peptone, casaminoacids or any combination of two or more of the foregoing.

According to some embodiments, the carbon substrate is selected from the group consisting of C5 sugars (such as arabinose or xylose), C6 sugars (such as glucose or fructose), lactose, sucrose, glycerol, glycerine, acetate, Corn steep liquor, yeast extract, component from a yeast extract, peptone, casaminoacids or combinations thereof.

In cases where the bacterium of the present invention is a methylotrophic bacterium, the culture medium preferably comprises a reduced one-carbon compound, such as methanol or methylamine, or a multi-carbon compound that contain no carbon-carbon bonds, such as dimethylamine. Thus, according to some embodiments, the culture medium comprises methanol as a carbon source. The concentration of methanol in the culture medium may generally be in the range from about 0,5% w/v to about 4 % w/v, such as from about 2% w/v to about 4 % w/v. According to some embodiments, the concentration of methanol in the culture medium is in the range from about 2,5% w/v to about 3,5% w/v.

As the nitrogen source, various ammonium salts such as ammonia and ammonium sulfate, other nitrogen compounds such as amines, a natural nitrogen source such as peptone, soybean-hydrolysate, and digested fermentative microorganism can be used. As minerals, potassium monophosphate, magnesium sulfate, sodium chloride, ferrous sulfate, manganese sulfate, calcium chloride, and the like can be used.

Suitably, the bacterium is cultivated under suitable conditions for the production of the desired product. Suitable conditions for culturing the respective bacterium are well known to the skilled person. Typically, a bacterium is cultured at a temperature ranging from about 20 to about 45°C, such as from about 30 to about 38°C, such as at about 37°C. The cultivation can be preferably performed under aerobic conditions, such as by a shaking culture, by a stirring culture or in a bioreactor with aeration, at a temperature of about 20 to about 45 °C, such as about 30 to 38 °C, preferably at about 37°C. The pH of the culture is usually above 5, such as in a range from about 6 to about 8, preferably from about 6.5 to about 7.5, more preferably from about 6.8 to about 7.2. The pH of the culture can be adjusted with ammonia, calcium carbonate, various acids, various bases, and buffers. The cultivation may be carried out for a period in the range from 10 to 70 h, preferably in a range from 24 to 60 h, more preferably in a range from 36 to 50 h.

After cultivation, solids such as cells can be removed from the culture medium by centrifugation or membrane filtration. The biochemical compound can be collected by conventional method for isolation and purification chemical compounds from a medium. Well-known purification procedures include, but are not limited to, centrifugation or filtration, precipitation, ion exchange, chromatographic methods such as e.g. ion exchange chromatography or gel filtration chromatography, and crystallization methods. The method may further comprise collecting the biochemical compound from the culture medium.

The biochemical compound produced by the method of the present invention may be any desired compound obtained through the cultivation of a bacterium of the present invention. Non-limiting examples include organic acids, amino acids, fatty acids and derivatives thereof. Non-limiting examples of organic acids include fumarate, glycolate, succinate, malate, malonate, lactates and derivatives thereof. Non-limiting examples of amino acids include glutamate, lysine, methionine, tryptophan, phenylalanine and derivatives thereof.

Generally, when employing a bacterium of the present invention in the production of a biochemical compound, the bacterium will have the ability to produce said biochemical compound. This means that the bacterium is able to produce, excrete or secrete, and/or cause accumulation of a biochemical compound of interest in a culture medium or in the bacterium when the bacterium is cultured in the medium. A bacterium is considered as having the ability to produce the biochemical compound of interest if it expresses all enzymes involved in the biosynthetic pathway resulting in the biochemical compound. The bacterium may inherently have the ability to produce the biochemical compound of interest or may be modified to have the ability to produce the biochemical compound of interest by using, e.g., DNA recombination techniques. In the latter case, the bacterium will be genetically modified to heterologously express the enzyme(s) required for the biosynthesis of the biochemical compound.

By way of example, if the biochemical compound to be produced is GABA or a derivative thereof, then the bacterium will have the ability to produce GABA. The bacterium is considered as having the ability to produce GABA or a derivative thereof if it expresses all enzymes involved in the biosynthetic pathway resulting in GABA or the derivative.

To produce GABA, most GABA-producing bacteria use the glutamate decarboxylase (GAD) enzyme, which converts glutamate to GABA through the removal of CO2. Thus, a bacterium having the ability to produce GABA suitably comprises (e.g., expresses) a polypeptide having glutamate decarboxylase (GAD) activity (EC 4.1.1.15; encoded by the gene *gadA* or *gadB* or orthologs thereof). The bacterium may inherently have the ability to produce GABA or a derivative thereof or may be modified to have the ability to produce GABA or a derivative thereof by using, e.g., DNA recombination techniques. In the latter case, the bacterium will be genetically modified to heterologously express the polypeptide having glutamate decarboxylase (GAD) activity.

As used herein, a "polypeptide having glutamate decarboxylase (GAD) activity" or a "polypeptide which has glutamate decarboxylase (GAD) activity" means a polypeptide that catalyzes the reaction: L-glutamate <=> 4-aminobutanoate + CO(2) (EC 4.1.1.15). Non-limiting examples of such polypeptide are provided in SEQ ID NOs: 211 to 220 and variants thereof having at least 70%, such as at least 80%, at least 85%, at least 90%, at least 95% or at least 97%, sequence identity therewith.

Thus, according to some embodiments, the bacterium of the present invention expresses a polypeptide having glutamate decarboxylase (GAD) activity.

According to some embodiments, the bacterium of the present invention expresses a polypeptide having glutamate decarboxylase (GAD) activity, which comprises an amino acid sequence having at least 70%, such as at least 75%, sequence identity to any one of the amino acid sequences set forth in SEQ ID NOs 211 to 220. According to some embodiments, the bacterium of the present invention expresses a polypeptide having glutamate decarboxylase (GAD) activity, which comprises an amino acid sequence having at least 80%, such as at least 85%, sequence identity to any one of the amino acid sequences set forth in SEQ ID NOs 211 to 220. According to some embodiments, the bacterium of the present invention expresses a polypeptide having glutamate decarboxylase (GAD) activity, which comprises an amino acid sequence having at least 90%, such as at least 95%, sequence identity to any one of the amino acid sequences set forth in SEQ ID NOs 211 to 220.

In bacteria, the GAD enzyme participates in the acid stress response. When some bacteria are exposed to low pH, they increase expression of the gad gene. This leads to rapid decarboxylation of glutamate, which consumes intracellular protons and increases the pH. GABA is produced in the process. Since GAD activity is required in acidic environments, it shows optimal activity at pH 4.0-5.0, but is inactive at neutral pH 7.0. The lack of GAD activity under normal physiological conditions of the cytosol, in particular neutral pH, prevents continuous GABA production at neutral pH.

Thus, while the present invention contemplates employing a wild type polypeptide which has glutamate decarboxylase (GAD) activity (i.e. a glutamate decarboxylase naturally found in an organism), it may be advantageous to employ mutant GAD enzymes, specially ones which are catalytically active at a pH in the range of pH 6 to pH 8.

Two approaches have been used to improve the pH range of GAD enzymes. One method removed part of the C-terminus of GAD, which physically blocks the catalytic site of GAD when the environmental pH is neutral. By truncating the C-terminus of the Lactobacillus brevis and Lactobacillus plantarum GAD enzyme, the pH range of the enzyme was expanded to 4.0-8.0 (Yu et al., 2012). In the second approach, specific sites on the GAD enzymes were mutated via random mutagenesis to obtain mutant enzyme active at neutral pH. By mutating the penultimate His residue of the E. coli GAD (H465), the C-terminus was again unable to block GAD activity at neutral pH (Pennacchieti, et al., 2009). An E89Q mutation in E. coli GAD also prevents the loss of activity at neutral pH (Thu Ho et al., 2013). A similar effect was observed in Lactobacillus brevis GAD where four amino acid changes were introduced (T17I, D294G, E312S, Q346H).

Thus, according to some embodiments, the bacterium of the present invention expresses a polypeptide which has glutamate decarboxylase (GAD) activity and shows catalytic activity at a pH in the range of pH 6 to pH 8, more preferably at pH 7.0.

According to some embodiments, the polypeptide having glutamate decarboxylase (GAD) activity is a GAD mutant comprising in its amino acid sequence at least one amino acid substitution compared to the wild type GAD enzyme from which it is derived rending the mutant active at a pH in the range of pH 6 to pH 8.

According to some embodiments, the at least one amino acid substitution is in a position corresponding to position 89 and/or position 465 of SEQ ID NO: 211. Thus, according to some embodiments, the GAD mutant comprises in its amino acid sequence an amino acid substitution in a position corresponding to position 89 of SEQ ID NO: 211. According to some embodiments, the GAD mutant comprises in its amino acid sequence an amino acid substitution in a position corresponding to position 465 of SEQ ID NO: 211. According to some embodiments, the GAD mutant comprises in its amino acid sequence two amino acid substitutions in positions corresponding to position 89 and position 465 of SEQ ID NO: 211.

According to some embodiments, the polypeptide is a GAD mutant having 2 to 20 amino acids, such as 2 to 14 amino acids, deleted from the C-terminus compared to the wild type GAD enzyme from which it is derived.

According to some embodiments, the polypeptide having glutamate decarboxylase (GAD) activity is selected from any one of the following:
a) a polypeptide having glutamate decarboxylase (GAD) activity which comprises an amino acid sequence having at least 70%, such as at least 80%, at least 85%, at least 90% or at least 95%, sequence identity with SEQ ID NO: 211 and comprising an amino acid substitution at a position corresponding to position 89 and/or position 465 in SEQ ID NO: 211;
b) a polypeptide having glutamate decarboxylase (GAD) activity which comprises an amino acid sequence having at least 70%, such as at least 80%, at least 85%, at least 90% or at least 95%, sequence identity with SEQ ID NO: 212 and comprising an amino acid substitution at a position corresponding to position 84 and/or position 457 in SEQ ID NO: 212;
c) a polypeptide having glutamate decarboxylase (GAD) activity which comprises an amino acid sequence having at least 70%, such as at least 80%, at least 85%, at least 90% or at least 95%, sequence identity with SEQ ID NO: 213 and comprising an amino acid substitution at a position corresponding to position 93 and/or position 467 in SEQ ID NO: 213;
d) a polypeptide having glutamate decarboxylase (GAD) activity which comprises an amino acid sequence having at least 70%, such as at least 80%, at least 85%, at least 90% or at least 95%, sequence identity with SEQ ID NO: 214 and comprising an amino acid substitution at a position corresponding to position 90 and/or position 467 in SEQ ID NO: 214;
e) a polypeptide having glutamate decarboxylase (GAD) activity which comprises an amino acid sequence having at least 70%, such as at least 80%, at least 85%, at least 90% or at least 95%, sequence identity with SEQ ID NO: 215 and comprising an amino acid substitution at a position corresponding to position 85 and/or position 462 in SEQ ID NO: 215;
f) a polypeptide having glutamate decarboxylase (GAD) activity which comprises an amino acid sequence having at least 70%, such as at least 80%, at least 85%, at least 90% or at least 95%, sequence identity with SEQ ID NO: 216 and comprising an amino acid substitution at a position corresponding to position 89 and/or position 465 in SEQ ID NO: 216;
g) a polypeptide having glutamate decarboxylase (GAD) activity which comprises an amino acid sequence having at least 70%, such as at least 80%, at least 85%, at least 90% or at least 95%, sequence identity with SEQ ID NO: 217 and comprising an amino acid substitution at a position corresponding to position 89 and/or position 466 in SEQ ID NO: 217;
h) a polypeptide having glutamate decarboxylase (GAD) activity which comprises an amino acid sequence having at least 70%, such as at least 80%, at least 85%, at least 90% or at least 95%, sequence identity with SEQ ID NO: 218 and comprising an amino acid substitution at a position corresponding to position 89 and/or position 466 in SEQ ID NO: 218;
i) a polypeptide having glutamate decarboxylase (GAD) activity which comprises an amino acid sequence having at least 70%, such as at least 80%, at least 85%, at least 90% or at least 95%, sequence identity with SEQ ID NO: 219 and comprising an amino acid substitution at a position corresponding to position 89 and/or position 463 in SEQ ID NO: 219;
j) a polypeptide having glutamate decarboxylase (GAD) activity which comprises an amino acid sequence having at least 70%, such as at least 80%, at least 85%, at least 90% or at least 95%, sequence identity with SEQ ID NO: 220 and comprising an amino acid substitution at a position corresponding to position 17, position 91, position 94, position 213, position 346 and/or position 468 in SEQ ID NO: 220; and
k) a polypeptide having glutamate decarboxylase (GAD) activity which comprises an amino acid sequence having at least 70%, such as at least 80%, at least 85%, at least 90% or at least 95%, sequence identity with SEQ ID NO: 211, 212, 213, 214, 215, 216, 217, 218, 219 or 220 wherein 2 to 20 amino acids, such as 2 to 14 amino acids, are deleted from the C-terminus.

According to some embodiments, the polypeptide having glutamate decarboxylase (GAD) activity comprises an amino acid sequence which has at least 70%, such as at least 75%, sequence identity with SEQ ID NO: 211 and comprises an amino acid substitution at a position corresponding to position 89 and/or position 465 in SEQ ID NO: 211. According to some embodiments, the polypeptide having glutamate decarboxylase (GAD) activity comprises an amino acid sequence which has at least 80%, such as at least 85%, sequence identity with SEQ ID NO: 211 and comprises an amino acid substitution at a position corresponding to position 89 and/or position 465 in SEQ ID NO: 211. According to some embodiments, the polypeptide having glutamate decarboxylase (GAD) activity comprises an amino acid sequence which has at least 90%, such as at least 95%, sequence identity with SEQ ID NO: 211 and comprises an amino acid substitution at a position corresponding to position 89 and/or position 465 in SEQ ID NO: 211.

According to some embodiments, the polypeptide having glutamate decarboxylase (GAD) activity comprises an amino acid sequence which has at least 70%, such as at least 75%, sequence identity with SEQ ID NO: 212 and comprises an amino acid substitution at a position corresponding to position 84 and/or position 457 in SEQ ID NO: 212. According to some embodiments, the polypeptide having glutamate decarboxylase (GAD) activity comprises an amino acid sequence which has at least 80%, such as at least 85%, sequence identity with SEQ ID NO: 212 and comprises an amino acid substitution at a position corresponding to position 84 and/or position 457 in SEQ ID NO: 212. According to some embodiments, the polypeptide having glutamate decarboxylase (GAD) activity comprises an amino acid sequence which has at least 90%, such as at least 95%, sequence identity with SEQ ID NO: 212 and comprises an amino acid substitution at a position corresponding to position 84 and/or position 457 in SEQ ID NO: 212.

According to some embodiments, the polypeptide having glutamate decarboxylase (GAD) activity comprises an amino acid sequence which has at least 70%, such as at least 75%, sequence identity with SEQ ID NO: 213 and comprises an amino acid substitution at a position corresponding to position 93 and/or position 467 in SEQ ID NO: 213. According to some embodiments, the polypeptide having glutamate decarboxylase (GAD) activity comprises an amino acid sequence which has at least 80%, such as at least 85%, sequence identity with SEQ ID NO: 213 and comprises an amino acid substitution at a position corresponding to position 93 and/or position 467 in SEQ ID NO: 213. According to some embodiments, the polypeptide having glutamate decarboxylase (GAD) activity comprises an amino acid sequence which has at least 90%, such as at least 95%, sequence identity with SEQ ID NO: 213 and comprises an amino acid substitution at a position corresponding to position 93 and/or position 467 in SEQ ID NO: 213.

According to some embodiments, the polypeptide having glutamate decarboxylase (GAD) activity comprises an amino acid sequence which has at least 70%, such as at least 75%, sequence identity with SEQ ID NO: 214 and comprising an amino acid substitution at a position corresponding to position 90 and/or position 467 in SEQ ID NO: 214. According to some embodiments, the polypeptide having glutamate decarboxylase (GAD) activity comprises an amino acid sequence which has at least 80%, such as at least 85%, sequence identity with SEQ ID NO: 214 and comprising an amino acid substitution at a position corresponding to position 90 and/or position 467 in SEQ ID NO: 214. According to some embodiments, the polypeptide having glutamate decarboxylase (GAD) activity comprises an amino acid sequence which has at least 90%, such as at least 95%, sequence identity with SEQ ID NO: 214 and comprising an amino acid substitution at a position corresponding to position 90 and/or position 467 in SEQ ID NO: 214.

According to some embodiments, the polypeptide having glutamate decarboxylase (GAD) activity comprises an amino acid sequence which has at least 70%, such as at least 75%, sequence identity with SEQ ID NO: 215 and comprises an amino acid substitution at a position corresponding to position 85 and/or position 462 in SEQ ID NO: 215. According to some embodiments, the polypeptide having glutamate decarboxylase (GAD) activity comprises an amino acid sequence which has at least 80%, such as at least 85%, sequence identity with SEQ ID NO: 215 and comprises an amino acid substitution at a position corresponding to position 85 and/or position 462 in SEQ ID NO: 215. According to some embodiments, the polypeptide having glutamate decarboxylase (GAD) activity comprises an amino acid sequence which has at least 90%, such as at least 95%, sequence identity with SEQ ID NO: 215 and comprises an amino acid substitution at a position corresponding to position 85 and/or position 462 in SEQ ID NO: 215.

According to some embodiments, the polypeptide having glutamate decarboxylase (GAD) activity comprises an amino acid sequence which has at least 70%, such as at least 75%, sequence identity with SEQ ID NO: 216 and comprises an amino acid substitution at a position corresponding to position 89 and/or position 465 in SEQ ID NO: 216. According to some embodiments, the polypeptide having glutamate decarboxylase (GAD) activity comprises an amino acid sequence which has at least 80%, such as at least 85%, sequence identity with SEQ ID NO: 216 and comprises an amino acid substitution at a position corresponding to position 89 and/or position 465 in SEQ ID NO: 216. According to some embodiments, the polypeptide having glutamate decarboxylase (GAD) activity comprises an amino acid sequence which has at least 90%, such as at least 95%, sequence identity with SEQ ID NO: 216 and comprises an amino acid substitution at a position corresponding to position 89 and/or position 465 in SEQ ID NO: 216.

According to some embodiments, the polypeptide having glutamate decarboxylase (GAD) activity comprises an amino acid sequence which has at least 70%, such as at least 75%, sequence identity with SEQ ID NO: 217 and comprises an amino acid substitution at a position corresponding to position 89 and/or position 466 in SEQ ID NO: 217. According to some embodiments, the polypeptide having glutamate decarboxylase (GAD) activity comprises an amino acid sequence which has at least 80%, such as at least 85%, sequence identity with SEQ ID NO: 217 and comprises an amino acid substitution at a position corresponding to position 89 and/or position 466 in SEQ ID NO: 217. According to some embodiments, the polypeptide having glutamate decarboxylase (GAD) activity comprises an amino acid sequence which has at least 90%, such as at least 95%, sequence identity with SEQ ID NO: 217 and comprises an amino acid substitution at a position corresponding to position 89 and/or position 466 in SEQ ID NO: 217.

According to some embodiments, the polypeptide having glutamate decarboxylase (GAD) activity comprises an amino acid sequence which has at least 70%, such as at least 75%, sequence identity with SEQ ID NO: 218 and comprises an amino acid substitution at a position corresponding to position 89 and/or position 466 in SEQ ID NO: 218. According to some embodiments, the polypeptide having glutamate decarboxylase (GAD) activity comprises an amino acid sequence which has at least 80%, such as at least 85%, sequence identity with SEQ ID NO: 218 and comprises an amino acid substitution at a position corresponding to position 89 and/or position 466 in SEQ ID NO: 218. According to some embodiments, the polypeptide having glutamate decarboxylase (GAD) activity comprises an amino acid sequence which has at least 90%, such as at least 95%, sequence identity with SEQ ID NO: 218 and comprises an amino acid substitution at a position corresponding to position 89 and/or position 466 in SEQ ID NO: 218.

According to some embodiments, the polypeptide having glutamate decarboxylase (GAD) activity comprises an amino acid sequence which has at least 70%, such as at least 75%, sequence identity with SEQ ID NO: 219 and comprises an amino acid substitution at a position corresponding to position 89 and/or position 463 in SEQ ID NO: 219. According to some embodiments, the polypeptide having glutamate decarboxylase (GAD) activity comprises an amino acid sequence which has at least 80%, such as at least 85%, sequence identity with SEQ ID NO: 219 and comprises an amino acid substitution at a position corresponding to position 89 and/or position 463 in SEQ ID NO: 219. According to some embodiments, the polypeptide having glutamate decarboxylase (GAD) activity comprises an amino acid sequence which has at least 90%, such as at least 95%, sequence identity with SEQ ID NO: 219 and comprises an amino acid substitution at a position corresponding to position 89 and/or position 463 in SEQ ID NO: 219.

According to some embodiments, the polypeptide having glutamate decarboxylase (GAD) activity comprises an amino acid sequence which has at least 70%, such as at least 75%, sequence identity with SEQ ID NO: 220 and comprises an amino acid substitution at a position corresponding to position 17, position 91, position 94, position 213, position 346 and/or position 468 in SEQ ID NO: 220. According to some embodiments, the polypeptide having glutamate decarboxylase (GAD) activity comprises an amino acid sequence which has at least 80%, such as at least 85%, sequence identity with SEQ ID NO: 1220 and comprises an amino acid substitution at a position corresponding to position 17, position 91, position 94, position 213, position 346 and/or position 468 in SEQ ID NO: 220. According to some embodiments, the polypeptide having glutamate decarboxylase (GAD) activity comprises an amino acid sequence which has at least 90%, such as at least 95%, sequence identity with SEQ ID NO: 220 and comprises an amino acid substitution at a position corresponding to position 17, position 91, position 94, position 213, position 346 and/or position 468 in SEQ ID NO: 220.

According to some embodiments, the polypeptide having glutamate decarboxylase (GAD) activity comprises an amino acid sequence having at least 70%, such as at least 75%, sequence identity with SEQ ID NO: 211, 212, 213, 214, 215, 216, 217, 218, 219 or 220 wherein 2 to 20 amino acids are deleted from the C-terminus. According to some embodiments, the polypeptide having glutamate decarboxylase (GAD) activity comprises an amino acid sequence having at least 80%, such as at least 85%, sequence identity with SEQ ID NO: 211, 212, 213, 214, 215, 216, 217, 218, 219 or 220 wherein 2 to 20 amino acids are deleted from the C-terminus. According to some embodiments, the polypeptide having glutamate decarboxylase (GAD) activity comprises an amino acid sequence having at least 90%, such as at least 95%, sequence identity with SEQ ID NO: 211, 212, 213, 214, 215, 216, 217, 218, 219 or 220, wherein 2 to 20 amino acids are deleted from the C-terminus.

According to some embodiments, the polypeptide having glutamate decarboxylase (GAD) activity comprises an amino acid sequence having at least 70%, such as at least 75%, sequence identity with SEQ ID NO: 211, 212, 213, 214, 215, 216, 217, 218, 219 or 220 wherein 2 to 14 amino acids are deleted from the C-terminus. According to some embodiments, the polypeptide having glutamate decarboxylase (GAD) activity comprises an amino acid sequence having at least 80%, such as at least 85%, sequence identity with SEQ ID NO: 211, 212, 213, 214, 215, 216, 217, 218, 219 or 220 wherein 2 to 14 amino acids are deleted from the C-terminus. According to some embodiments, the polypeptide having glutamate decarboxylase (GAD) activity comprises an amino acid sequence having at least 90%, such as at least 95%, sequence identity with SEQ ID NO: 211, 212, 213, 214, 215, 216, 217, 218, 219 or 220, wherein 2 to 14 amino acids are deleted from the C-terminus.

According to some embodiments, the polypeptide having glutamate decarboxylase (GAD) activity comprises an amino acid sequence having at least 70%, such as at least 75%, sequence identity with SEQ ID NO: 211, 212, 213, 214, 215, 216, 217, 218, 219 or 220 wherein 4 to 10 amino acids are deleted from the C-terminus. According to some embodiments, the polypeptide having glutamate decarboxylase (GAD) activity comprises an amino acid sequence having at least 80%, such as at least 85%, sequence identity with SEQ ID NO: 211, 212, 213, 214, 215, 216, 217, 218, 219 or 220 wherein 4 to 10 amino acids are deleted from the C-terminus. According to some embodiments, the polypeptide having glutamate decarboxylase (GAD) activity comprises an amino acid sequence having at least 90%, such as at least 95%, sequence identity with SEQ ID NO: 211, 212, 213, 214, 215, 216, 217, 218, 219 or 220, wherein 4 to 10 amino acids are deleted from the C-terminus.

According to some embodiments, the polypeptide having glutamate decarboxylase (GAD) activity comprises an amino acid sequence having at least 70%, such as at least 75%, sequence identity with SEQ ID NO: 221, with the proviso that the amino acid at position 89 is not E, preferably with the proviso that the amino acid at position 89 is Q. According to some embodiments, the polypeptide having glutamate decarboxylase (GAD) activity comprises an amino acid sequence having at least 80%, such as at least 85%, sequence identity with SEQ ID NO: 221, with the proviso that the amino acid at position 89 is not E, preferably with the proviso that the amino acid at position 89 is Q. According to some embodiments, the polypeptide having glutamate decarboxylase (GAD) activity comprises an amino acid sequence having at least 90%, such as at least 95%, sequence identity with SEQ ID NO: 221, with the proviso that the amino acid at position 89 is not E, preferably with the proviso that the amino acid at position 89 is Q.

According to some embodiments, the polypeptide having glutamate decarboxylase (GAD) activity comprises an amino acid sequence having at least 70%, such as at least 75%, sequence identity with SEQ ID NO: 223, with the proviso that the amino acid at position 84 is not E, preferably with the proviso that the amino acid at position 84 is Q. According to some embodiments, the polypeptide having glutamate decarboxylase (GAD) activity comprises an amino acid sequence having at least 80%, such as at least 85%, sequence identity with SEQ ID NO: 223, with the proviso that the amino acid at position 84 is not E, preferably with the proviso that the amino acid at position 84 is Q. According to some embodiments, the polypeptide having glutamate decarboxylase (GAD) activity comprises an amino acid sequence having at least 90%, such as at least 95%, sequence identity with SEQ ID NO: 223, with the proviso that the amino acid at position 84 is not E, preferably with the proviso that the amino acid at position 84 is Q.

According to some embodiments, the polypeptide having glutamate decarboxylase (GAD) activity comprises an amino acid sequence having at least 70%, such as at least 75%, sequence identity with SEQ ID NO: 225, with the proviso that the amino acid at position 17 is not T, the amino acid at position 294 is not D, the amino acid at position 312 is not E and /or the amino acid at position 346 is not Q, preferably with the proviso that the amino acid at position 17 is I, the amino acid at position 294 is G, the amino acid at position 312 is S and /or the amino acid at position 346 is H. According to some embodiments, the polypeptide having glutamate decarboxylase (GAD) activity comprises an amino acid sequence having at least 80%, such as at least 85%, sequence identity with SEQ ID NO: 225, with the proviso that the amino acid at position 17 is not T, the amino acid at position 294 is not D, the amino acid at position 213 is not E and /or the amino acid at position 346 is not Q, preferably with the proviso that the amino acid at position 17 is I, the amino acid at position 294 is G, the amino acid at position 312 is S and /or the amino acid at position 346 is H. According to some embodiments, the polypeptide having glutamate decarboxylase (GAD) activity comprises an amino acid sequence having at least 90%, such as at least 95%, sequence identity with SEQ ID NO: 225, with the proviso that the amino acid at position 17 is not T, the amino acid at position 294 is not D, the amino acid at position 312 is not E and /or the amino acid at position 346 is not Q, preferably with the proviso that the amino acid at position 17 is I, the amino acid at position 294 is G, the amino acid at position 312 is S and /or the amino acid at position 346 is H.

In order to achieve heterologous expression of the enzyme(s) required for the biosynthesis of the biochemical compound, such as GABA, one or more exogenous nucleic acid molecules which comprise(s) one or more nucleotide sequences encoding said enzyme(s) have been introduced in the bacterium. Thus, a genetically modified bacterium may comprise one or more exogenous nucleic acid molecules comprise(s) one or more nucleotide sequences encoding said enzyme(s) in question. In order to facilitate expression of said enzyme(s) in the bacterium, the exogenous nucleic acid molecule(s) may comprise suitable regulatory elements such as a promoter that is functional in the bacterial cell to cause the production of an mRNA molecule and that is operably linked to the nucleotide sequence(s) encoding said enzyme(s).

Techniques for introducing an exogenous nucleic acid molecule, such as a DNA molecule, into a bacterial cell are well-known to those of skill in the art, and include transformation (e.g., heat shock or natural transformation) among others.

Suitably, an exogenous nucleic acid molecule comprises at least one transcriptional unit comprising, from 5' to 3', a promoter that is functional in the bacterium to cause the production of an mRNA molecule and that is operably linked to a nucleotide sequence encoding said enzyme in question, and a transcriptional terminator sequence.

Promoters useful in accordance with the invention are any known promoters that are functional in a given host cell to cause the production of an mRNA molecule. Many such promoters are known to the skilled person. Such promoters include promoters normally associated with other genes, and/or promoters isolated from any bacteria. The use of promoters for protein expression is generally known to those of skilled in the art of molecular biology, for example, see Sambrook et al., Molecular cloning: A Laboratory Manual, Cold Spring Harbor Laboratory, Cold Spring Harbor, N.Y., 1989. The promoter employed may be inducible, such as a temperature inducible promoter (e.g., a pL or pR phage lambda promoters, each of which can be controlled by the temperature-sensitive lambda repressor c1857). The term "inducible" used in the context of a promoter means that the promoter only directs transcription of an operably linked nucleotide sequence if a stimulus is present, such as a change in temperature or the presence of a chemical substance ("chemical inducer"). As used herein, "chemical induction" according to the present invention refers to the physical application of an exogenous or endogenous substance (incl. macromolecules, e.g., proteins or nucleic acids) to a host cell. This has the effect of causing the target promoter present in the host cell to increase the rate of transcription. Alternatively, the promoter employed may be constitutive. The term "constitutive" used in the context of a promoter means that the promoter is capable of directing transcription of an operably linked nucleotide sequence in the absence of stimulus (such as heat shock, chemicals etc.).

Temperature induction systems work, for example, by employing promoters that are repressed by thermolabile repressors. These repressors are active at lower temperatures for example at 30°C, while unable to fold correctly at 37 °C and are therefore inactive. Such circuits therefore can be used to directly regulate the genes of interest (St-Pierre et al. 2013) also by genome integration of the genes along with the repressors. Examples of such as a temperature inducible expression system are based on the pL and/or pR λ phage promoters which are regulated by the thermolabile cl857 repressor. Similar to the genome integrated DE3 system, the expression of the T7 RNA polymerase gene may also be controlled using a temperature controlled promoter system (Mertens et al. 1995), while the expression of the genes of interest can be controlled using a T7 promoter.

Non-limiting examples of promoters functional in bacteria include both constitutive and inducible promoters such as T7 promoter, the beta-lactamase and lactose promoter systems; alkaline phosphatase (phoA) promoter, a tryptophan (trp) promoter system, tetracycline promoter, lambda-phage promoter, ribosomal protein promoters; and hybrid promoters such as the tac promoter. Other bacterial and synthetic promoters are also suitable.

Besides a promoter, the exogenous nucleic acid molecule may further comprise at least one regulatory element selected from a 5' untranslated region (5'UTR) and 3' untranslated region (3' UTR). Many such 5' UTRs and 3' UTRs derived from prokaryotes and eukaryotes are well known to the skilled person. Such regulatory elements include 5' UTRs and 3' UTRs normally associated with other genes, and/or 5' UTRs and 3' UTRs isolated from any bacteria.

Usually, the 5' UTR contains a ribosome binding site (RBS), also known as the Shine Dalgarno sequence which is usually 3-10 base pairs upstream from the initiation codon.

The exogenous nucleic acid molecule may be a DNA construct, such as an expression cassette or a vector. The exogenous nucleic acid molecule may thus be a vector, such as an expression vector, or part of such vector, such as an expression cassette comprised by such vector. Normally, such a vector remains extrachromosomal within the bacterial cell which means that it is found outside of the genome of the bacterial cell. Alternatively, the exogenous nucleic acid molecule may be stably integrated into the genome of the bacterium (e.g., by random or targeted insertion). Particularly, the exogenous nucleic acid molecule may be an expression cassette stably integrated into the genome of the bacterium (e.g., by random or targeted insertion).

The present invention also provides a biochemical compound obtainable by a method as detailed above.

The present invention also provides methods for producing biomass comprising cultivating a bacterium according to the invention under suitable culture conditions.

Details regarding the culture conditions, such as the culture medium, temperature etc., have been given above in connection with the method of producing a biochemical, and apply *mutatis mutandis.*

### Certain other definitions

As used herein, a "biochemical compound" means any carbon-based compound that is produced by a living organism.

The term "mesophilic" as used herein in the context of a bacterium means that the bacterium grows best in moderate temperature with an optimum growth range from 20 to 45 °C.

The term "methylotrophic" as used herein in the context of a bacterium means that the bacterium can use reduced one-carbon compounds, such as methanol, methane, formate, or methylamine, as the carbon source for their growth, and multi-carbon compounds that contain no carbon-carbon bonds, such as dimethyl ether and dimethylamine.

"Polypeptide" and "protein" are used interchangeably herein to denote a polymer of at least two amino acids covalently linked by an amide bond, regardless of length or post-translational modification (e.g., glycosylation, phosphorylation, lipidation, myristoylation, ubiquitination, etc.). Included within this definition are D- and L-amino acids, and mixtures of D- and L-amino acids.

"Nucleic acid" or "polynucleotide" are used interchangeably herein to denote a polymer of at least two nucleic acid monomer units or bases (e.g., adenine, cytosine, guanine, thymine) covalently linked by a phosphodiester bond, regardless of length or base modification.

"Recombinant" or "non-naturally occurring" when used with reference to, e.g., a host cell, nucleic acid, or polypeptide, refers to a material, or a material corresponding to the natural or native form of the material, that has been modified in a manner that would not otherwise exist in nature, or is identical thereto but produced or derived from synthetic materials and/or by manipulation using recombinant techniques. Non-limiting examples include, among others, recombinant bacterial cells expressing genes that are not found within the native (non-recombinant) form of the cell or express native genes that are otherwise expressed at a different level.

"Heterologous" or "exogenous" as used herein in the context of a gene or nucleic acid molecule refer to a gene or nucleic acid molecule (i.e. DNA or RNA molecule) that does not occur naturally as part of the genome of the bacterium in which it is present or which is found in a location or locations in the genome that differ from that in which it occurs in nature. Thus, a "heterologous" or "exogenous" gene or nucleic acid molecule is not endogenous to the bacterium and has been exogenously introduced into the microorganism. A "heterologous" gene or nucleic acid molecule DNA molecule may be from a different organism, a different species, a different genus or a different kingdom, as the host DNA.

"Heterologous" as used herein in the context of a polypeptide means that a polypeptide is normally not found in or made (i.e. expressed) by the host microorganism, but derived from a different organism, a different species, a different genus or a different kingdom.

As used herein, the term "ortholog" or "orthologs" refers to genes, nucleic acid molecules encoded thereby, i.e., mRNA, or proteins encoded thereby that are derived from a common ancestor gene but are present in different species.

By "decreased expression" of a gene it is meant that the amount of the transcription product, respectively the amount of the polypeptide (e.g., enzyme) encoded by said gene produced by the modified bacterium is decreased compared to an otherwise identical bacterium that does not carry said modification. More particularly, by "decreased expression" of a gene it is meant that the amount of the transcription product, respectively the amount of the polypeptide (e.g., enzyme) encoded by said gene produced by the modified bacterium is decreased by at least 10%, such as at least 20%, at least 30%, at least 40%, at least 50% at least 60%, at least 70%, at least 80%, at least 90% or at least 100%, compared to an otherwise identical bacterium that does not carry said modification. The level of expression of a gene can be determined by well-known methods, including PCR, Southern blotting, and the like. In addition, the level of gene expression can be estimated by measuring the amount of mRNA transcribed from the gene using various well-known methods, including Northern blotting, quantitative RT-PCR, and the like. The amount of the polypeptide encoded by the gene can be measured by well-known methods, including ELISA, Immunohistochemistry or Western Blotting and the like.

Expression of a gene can be decreased by introducing a mutation into the gene in the genome of the bacterium so that the intracellular activity of the polypeptide encoded by the gene is decreased as compared to an otherwise identical bacterium that does not carry said mutation. Mutations which result in a decreased expression of the gene include the replacement of one nucleotide or more to cause an amino acid substitution in the polypeptide encoded by the gene (missense mutation), introduction of a stop codon (nonsense mutation), deletion or insertion of nucleotides to cause a frame shift, insertion of a drug-resistance gene, or deletion of a part of the gene or the entire gene (Qiu and Goodman, 1997; Kwon et al., 2000). Expression can also be decreased by modifying an expression regulating sequence such as the promoter, the Shine-Dalgarno (SD) sequence, etc. Expression of the gene can also be decreased by gene replacement (Datsenko and Wanner, 2000), such as the "lambda-red mediated gene replacement". The lambda-red mediated gene replacement is a particularly suitable method to inactive one or more genes as described herein.

"Inactivating", "inactivation" and "inactivated", when used in the context of a gene or gene cluster, means that the gene or gene cluster in question no longer expresses a functional protein. It is possible that the modified DNA region is unable to naturally express the gene or gene cluster due to the deletion of a part of or the entire sequence of the gene or gene cluster, the shifting of the reading frame of the gene or gene cluster, the introduction of missense/nonsense mutation(s), or the modification of the regulatory region of the gene or gene cluster, including sequences controlling gene expression, such as a promoter, enhancer, attenuator, ribosome- binding site, etc. Preferably, a gene or gene cluster of interest is inactivated by deletion of a part of or the entire sequence of the gene or gene cluster, such as by gene replacement. Inactivation may also be accomplished by introducing or expressing a rare-cutting endonuclease able to selectively inactivating by DNA cleavage, preferably by double-strand break, the gene or gene cluster of interest. A "rare-cutting endonuclease" within the context of the present invention includes transcription activator-like effector (TALE) nucleases, meganucleases, zing-finger nucleases (ZFN), and RNA-guided endonucleases.

The presence or absence of a gene or gene cluster in the genome of a bacterium can be detected by well-known methods, including PCR, Southern blotting, and the like. In addition, the level of gene expression can be estimated by measuring the amount of mRNA transcribed from the gene or gene cluster using various well-known methods, including Northern blotting, quantitative RT-PCR, and the like. The amount of the polypeptide encoded by the gene or gene cluster can be measured by well-known methods, including SDS-PAGE followed by an immunoblotting assay (Western blotting analysis), and the like.

As used herein, "decreased", "decreasing" or "decrease of" expression of a polypeptide (such as a polypeptide as described herein) means that the expression of said polypeptide in a modified bacterium is reduced compared to the expression of said polypeptide in an otherwise identical bacterium that does not carry said modification (control). The expression of a polypeptide in a modified bacterium may be reduced by at least about 10 %, and preferably by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the expression of said polypeptide in an otherwise identical bacterium that does not carry said modification (control). More particularly, "decreased", "decreasing" or "decrease of" expression of a polypeptide means that the amount of the polypeptide in the modified bacterium is reduced by at least about 10 %, and preferably by at least about 20%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the amount of said polypeptide in an otherwise identical bacterium that does not carry said modification (control). The expression or amount of a polypeptide in a bacterium can be determined by any suitable means know in the art, including techniques such as ELISA, Immunohistochemistry, Western Blotting or Flow Cytometry.

As used herein, "abolished" expression of a polypeptide (such as a polypeptide as described herein) means that the expression of said polypeptide in a modified bacterium is not detectable compared to the expression of said polypeptide in an otherwise identical bacterium that does not carry said modification (control).

As used herein, "decreased", "decreasing" or "decrease of" activity of a polypeptide (such as an enzyme as described herein) means that the catalytic activity of said polypeptide in a modified bacterium is reduced compared to the catalytic activity of said polypeptide in an otherwise identical bacterium that does not carry said modification (control). The activity of a polypeptide in a modified bacterium may be reduced by at least about 10 %, and preferably by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 99% or 100%, or any percentage, in whole integers between 10% and 100% (e.g., 6%, 7%, 8%, etc.), compared to the expression of said polypeptide in an otherwise identical bacterium that does not carry said modification (control). The activity of a polypeptide in a bacterium can be determined by any suitable protein and enzyme activity assay.

"Expression" includes any step involved in the production of a polypeptide (e.g., encoded enzyme) including, but not limited to, transcription, post-transcriptional modification, translation, post-translational modification, and secretion.

As used herein, "regulatory region" of a gene or gene cluster refers to a nucleic acid sequence that affect the expression of a coding sequence. Regulatory regions are known in the art and include, but are not limited to, promoters, enhancers, transcription terminators, polyadenylation sites, matrix attachment regions and/or other elements that regulate expression of a coding sequence.

"Substitution" or "substituted" refers to modification of the polypeptide by replacing one amino acid residue with another, for instance the replacement of an Serine residue with a Glycine or Alanine residue in a polypeptide sequence is an amino acid substitution. When used with reference to a polynucleotide, "substitution" or "substituted" refers to modification of the polynucleotide by replacing one nucleotide with another, for instance the replacement of a cytosine with a thymine in a polynucleotide sequence is a nucleotide substitution.

"Conservative substitution", when used with reference to a polypeptide, refers to a substitution of an amino acid residue with a different residue having a similar side chain, and thus typically involves substitution of the amino acid in the polypeptide with amino acids within the same or similar class of amino acids. By way of example and not limitation, an amino acid with an aliphatic side chain may be substituted with another aliphatic amino acid, e.g., alanine, valine, leucine, and isoleucine; an amino acid with hydroxyl side chain is substituted with another amino acid with a hydroxyl side chain, e.g., serine and threonine; an amino acid having an aromatic side chain is substituted with another amino acid having an aromatic side chain, e.g., phenylalanine, tyrosine, tryptophan, and histidine; an amino acid with a basic side chain is substituted with another amino acid with a basic side chain, e.g., lysine and arginine; an amino acid with an acidic side chain is substituted with another amino acid with an acidic side chain, e.g., aspartic acid or glutamic acid; and a hydrophobic or hydrophilic amino acid is replaced with another hydrophobic or hydrophilic amino acid, respectively.

"Non-conservative substitution", when used with reference to a polypeptide, refers to a substitution of an amino acid in a polypeptide with an amino acid with significantly differing side chain properties. Non-conservative substitutions may use amino acids between, rather than within, the defined groups and affects (a) the structure of the peptide backbone in the area of the substitution (e.g., serine for glycine), (b) the charge or hydrophobicity, or (c) the bulk of the side chain. By way of example and not limitation, an exemplary non-conservative substitution can be an acidic amino acid substituted with a basic or aliphatic amino acid; an aromatic amino acid substituted with a small amino acid; and a hydrophilic amino acid substituted with a hydrophobic amino acid.

As used herein, "vector" refers to a nucleic acid molecule capable of transporting another nucleic acid molecule to which it has been linked. One type of vector is a "plasmid", which refers to a circular double stranded nucleic acid loop into which additional nucleic acid segments can be ligated. Certain vectors are capable of directing the expression of genes to which they are operatively linked. Such vectors are referred to herein as "expression vectors". Certain other vectors are capable of facilitating the insertion of an exogenous nucleic acid molecule into a genome of a bacterium. Such vectors are referred to herein as "transformation vectors". In general, vectors of utility in recombinant nucleic acid techniques are often in the form of plasmids. In the present specification, "plasmid" and "vector" can be used interchangeably as the plasmid is the most commonly used form of a vector. Large numbers of suitable vectors are known to those of skill in the art and commercially available.

As used herein, "promoter" refers to a sequence of DNA, usually upstream (5') of the coding region of a structural gene, which controls the expression of the coding region by providing recognition and binding sites for RNA polymerase and other factors which may be required for initiation of transcription. The selection of the promoter will depend upon the nucleic acid sequence of interest. A suitable "promoter" is generally one which is capable of supporting the initiation of transcription in a bacterium of the invention, causing the production of an mRNA molecule.

As used herein, "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequence. A promoter sequence is "operably-linked" to a gene when it is in sufficient proximity to the transcription start site of a gene to regulate transcription of the gene.

"Percentage of sequence identity," "% sequence identity" and "percent identity" refers to sequence identity between a nucleotide sequence and a reference nucleotide sequence or between an amino acid sequence and a reference amino acid sequence. Sequence identity can be determined by comparing a position in each sequence which may be aligned for purposes of comparison. When a position in the compared sequence is occupied by the same base or amino acid, then the molecules are identical at that position. A degree of identity between nucleotide or amino acid sequences is a function of the number of identical or matching nucleotides or amino acids at positions shared by the nucleotide or amino acid sequences, respectively. Various alignment algorithms and/or programs may be used to calculate the identity between two sequences, including FASTA or BLAST which are available as a part of the GCG sequence analysis package (University of Wisconsin, Madison, Wis.), and can be used with default settings.

"Reference sequence" or "reference amino acid sequence" refers to a defined sequence to which another sequence is compared. In the context of the present invention a reference amino acid sequence may, for example, be an amino acid sequence set forth in SEQ ID NO: 1.

As used herein, "derivative" of GABA refers to a compound derived from GABA by modification thereof. Non-limiting examples of a GABA "derivative" include 2-pyrrolidone, N-methyl-2-pyrrolidone and polybutyrolactam (Nylon 4).

As used herein, the term "about" means plus or minus 10% of the numerical value of the number with which it is being used.

Where a numerical limit or range is stated herein, the endpoints are included. Also, all values and sub ranges within a numerical limit or range are specifically included as if explicitly written out.

As used herein, the indefinite articles "a" and "an" mean "at least one" or "one or more" unless the context clearly dictates otherwise.

As used herein, the terms "comprising", "including", "having" and grammatical variants thereof are to be taken as specifying the stated features, steps or components but do not preclude the addition of one or more additional features, steps, components or groups thereof.

Having generally described this invention, a further understanding can be obtained by reference to certain specific examples, which are provided herein for purposes of illustration only, and are not intended to be limiting unless otherwise specified.

### Examples

### Example 1: Identification of polyphosphate kinase (ppk) gene in Methylobacilli

Wild-type *Methylobacilli* were found to consume much higher amounts of phosphate than would be required for biomass formation according to the standard biomass formula. Polyphosphate was identified as a potential phosphate sink and the presence of intracellular polyphosphate granules was confirmed with fluorescent microscopy and poly-phosphate specific staining with the DAPI fluorescent dye. Synthesis and degradation of polyP in microorganisms is catalyzed polyphosphate kinase enzymes (ppk). To identify the gene responsible for polyp formation in *Methylobacillus* a known ppk gene sequence from *Escherichia coli* (SEQ ID NO: 11) was used to perform a BLAST search on all available genomes from the *Methylobacillus* genus, which generated 10 hits (data not shown). The same was repeated on the *Methylobacterium* genus, yielding 176 hits (data not shown). A sequence alignment using the MAFT algorithm was performed using the SnapGene Version 6.0 software. Residues forming the active site (Zhu et al. 2005) and residues necessary for poly-phosphate synthesis activity in the *Escherichia coli* enzyme were identified from the literature (Tzeng and Kornberg, 2000). The alignment revealed that the crucial amino acids at positions corresponding to *Escherichia coli* ppk1 (SEQ ID NO: 11) R375, S380A, F488A, P507A, R564A, R621A, Q674A, as well as the active-site amino acids corresponding to *Escherichia coli* (SEQ ID NO: 11) amino acids H435 and H592 were conserved in over 95% of the analysed sequences (data not shown).

### Example 2: Deletion of polyphosphate kinase gene in Methylobacillus flagellatus

The polyphosphate kinase gene of *Methylobacillus flagellatus* (SEQ ID NO: 2) was deleted from the chromosome by homologous recombination of a selection marker-containing DNA construct into the original ppk gene locus. Briefly, a 2000 base pair sequence upstream of the ppk gene and a 2000 base pair sequence downstream of the gene were amplified from genomic DNA with PCR. The fragments were appended to an antibiotic resistance cassette, containing its own promoter, terminator and ribosome binding site. The resulting linear construct was transformed into the *Methylobacillus* strain by electroporation. A selection step on antibiotic containing, minimal medium agar plates with methanol as the carbon source was used to identify successful transformants. DNA integration into the correct locus was confirmed by colony PCR. The entire region after integration was amplified from the genome and checked by DNA sequencing. To ensure the polyphosphate gene was not present in the genome, a set of PCR primers was designed to target the gene specifically. Colony PCR utilizing these internal primers, that did not result in an amplified fragment was further evidence, that the ppk gene was no longer present.

### Example 3: Deletion of polyphosphate kinase gene in Methylobacillus glycogenes:

The polyphosphate kinase gene of *Methylobacillus glycogenes* (SEQ ID NO: 4) was deleted from the chromosome by homologous recombination of a selection marker-containing DNA construct into the original ppk gene locus. Briefly, a 2000 base pair sequence upstream of the ppk gene and a 2000 base pair sequence downstream of the gene were amplified from genomic DNA with PCR. The fragments were appended to a kanamycin-resistance cassette, containing its own promoter and terminator and the linear construct was transformed into the *Methylobacillus* strain by electroporation. A selection step on kanamycin containing, minimal methanol medium agar plates was used to identify successful transformants and DNA integration into the correct locus was confirmed by colony PCR. The entire region after integration was amplified from the genome and checked by DNA sequencing. To ensure the polyphosphate gene was not present in the genome, a set of PCR primers was designed to target the ppk gene specifically. A negative colony PCR utilizing the internal primers, in combination with the other evidence was used as an indication that the ppk gene was no longer present.

### Example 4: Comparison of polyP granules in WT and del-ppK strain

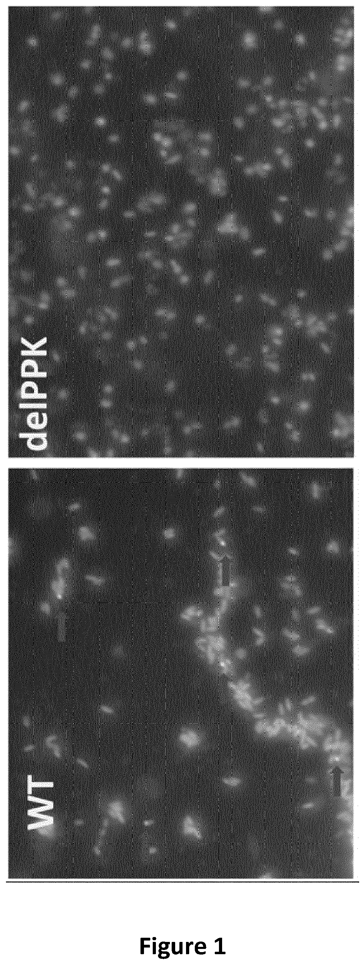

Cultures of *Methylobacillus flagellatus* with and without deletion of the ppK gene (SEQ ID NO: 2) were cultured in 5-liter bioreactors and shake-flasks in minimal methanol media. After sufficient cell density was achieved (OD600 ^{~}2), samples from each culture were stained with 40 micromolar 4,6-diamidino-2-phenylindole (DAPI). The staining solution was prepared in pH 7 phosphate-buffered saline. DAPI is commonly used as a DNA stain, but it can also be used to stain polyphosphate granules when added to the sample in higher concentrations (micromolar range). When bound to polyphosphate granules, the emission spectrum of the dye undergoes a spectral shift towards higher wavelengths, from blue to yellow. Samples were excited with ultraviolet (405 nm) light and observed through a long-pass 430 emission filter. Cells expressing the ppK gene were observed to contain polyphosphate granules, while cells in which the ppK gene was deleted contained no granules **(****Figure 1****)**

### Example 5: Comparison of fermentation with and without ppK disruption, production of biomass

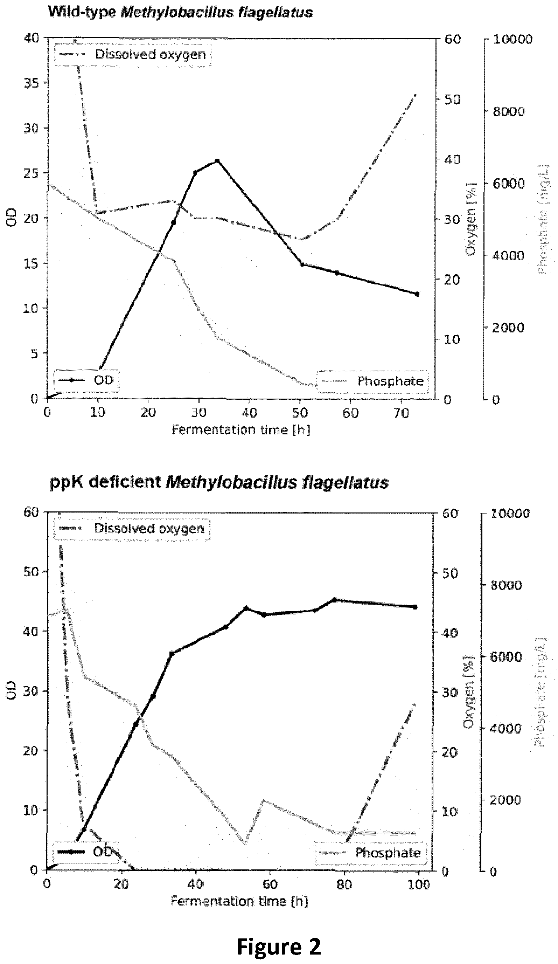

Side to side bioreactor fermentations were performed with *Methylobacillus flagellatus* strains with and without the ppK gene. A mineral medium containing KH₂PO₄, Na₂HPO₄, MgSO₄, NH₄SO₄ and a trace element mixture was used. Methanol concentration was dynamically maintained at 4-5 g/L throughout the fermentations via a feedback loop using an on-line proprietary methanol sensor. The pH was kept at 7 by automatic addition of ammonium hydroxide, which also served as source of nitrogen. Bioprocess parameters such as optical density, CFU counts, whole-broth dry weight and free phosphate were measured regularly. In the non-ppK deletion bioprocess, rapid cell lysis occurred once optical density reached ^{~}30, observed through a drop in optical density, a 99% decrease in colony forming units (CFU) and in the feeding rates of methanol and ammonium hydroxide. When the ppK gene was not present, optical density reached higher values of ^{~}45-50, CFU remained constant upon reaching stationary phase and no sharp drop-in feeding rates was observed, indicating cells remained metabolically active during stationary phase. Stationary phase could be maintained for multiple days, during which product synthesis can occur (**Figure 2**).

### Example 6: Comparison of GABA production with or without ppK deletion

Side to side bioreactor fermentations were performed with GABA-producing *Methylobacillus flagellatus* with or without the ppK gene. To enable GABA production in the strains, a glutamate dehydrogenase enzyme from *E. coli* (Eco GAD mutant E89Q ΔC14) was expressed (SEQ ID NO: 222), encoding the protein according to SEQ ID NO: 221. The expressed *E. coli* GAD enzyme contained the point mutation E89Q and was truncated at the C-terminus by removing the final 14 amino acids. These modifications were introduced to alleviate the pH-dependency of the native *E. coli* GAD enzyme and enable GABA synthesis at neutral pH. The genes were expressed from plasmid under the control of the IPTG-inducible lacO/trc promotor.

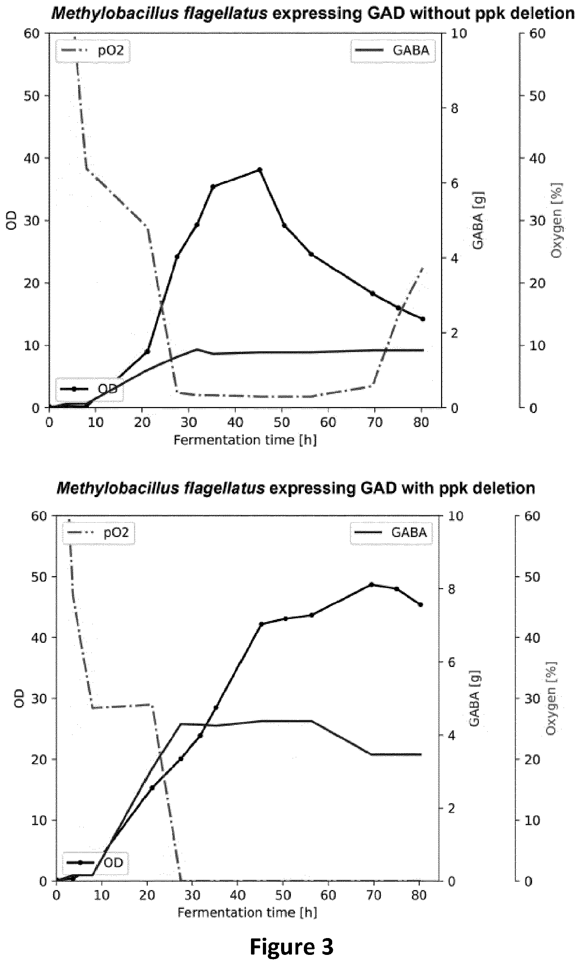

A mineral medium containing KH₂PO₄, Na₂HPO₄, MgSO₄, NH₄SO₄ and a trace element mixture was used. Methanol concentration was dynamically maintained at 4-5 g/L throughout the fermentations via a feedback loop using an on-line proprietary methanol sensor. The pH was kept at 7 by automatic addition of ammonium hydroxide, which also served as source of nitrogen. Samples from both bioreactors were taken in regular intervals and analysed for GABA content by HPLC. When the ppK gene is present cell lysis occurred when the culture reached an optical density of ^{~}30. This was followed by a spike of GABA production, as the expressed GAD enzyme along with other cellular contents was released into the fermentation broth. No further production of GABA was detected after this initial spike. In the case of the ppK deletion strain, GABA production was consistent throughout the process, accumulating during stationary phase **(****Figure 3****).**

### Example 7 - Bioinformatic analysis of Acyl-homoserine lactone synthase

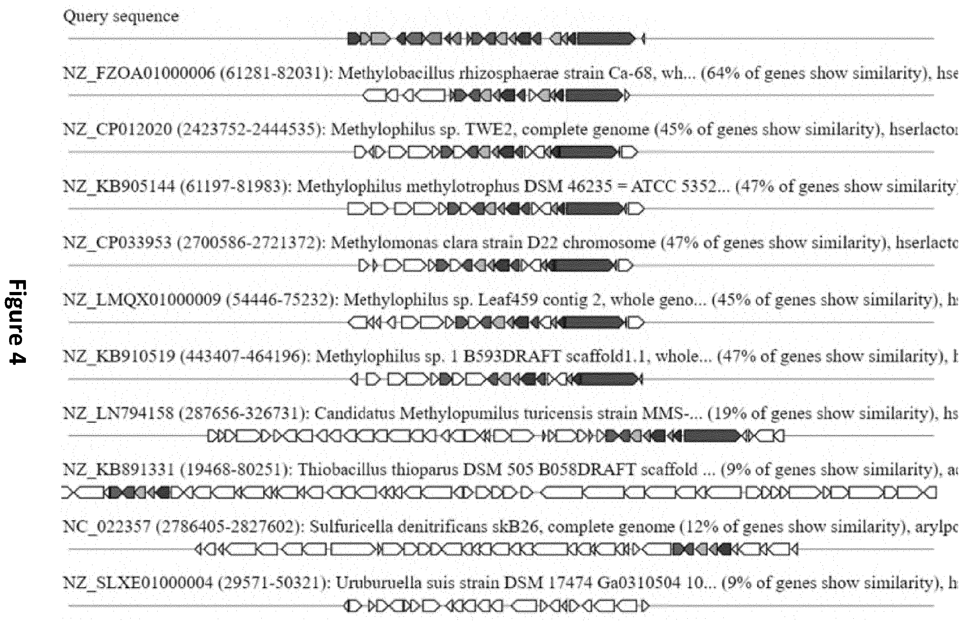

Programmed cell death has been linked to quorum-sensing mechanisms. In Gram-negative bacteria, quorum sensing depends on N-acyl homoserine lactones (AHL), which are synthesized by N-acyl homoserine lactone synthase enzymes. To search for the presence of a quorum sensing related gene cluster in *Methylobacillus flagellatus*, the antiSMASH 6.0 (Blin et al., 2021) algorithm was utilized to predict gene clusters, using the entire *Methylobacillus flagellatus* genome as the input (NCBI accession number: NC_007947.1). The algorithm attempted to map characterized gene clusters with known AHL synthesis function to the analysed genome **(****Figure 4****).** A single homoserine lactone production cluster was identified (SEQ ID NO: 227), including a homoserine lactone synthase gene (SEQ ID NO: 14). We then used BLAST to identify homologs of the *M. flagellatus* enzyme (SEQ ID NO: 13) in the genomes of *Methylobacillus flagellatus*, *Methylobacillus glycogenes, Methylobacillus rhizosphaerae, Methylobacterium organophilum, Methylorubrum extorquens and Methylorubrum extorquens_AM1.* We identified several candidate AHL synthase proteins in the organisms (SEQ ID NOs: 15, 17, 19, 21, 23 and 25, respectively).

### Example 8 - Deletion of AHL synthase gene in M. flagellatus:

The AHL synthase gene of *Methylobacillus flagellatus* (SEQ ID NO: 14) was deleted from the chromosome by homologous recombination of a selection marker-containing DNA construct into the original AHL synthase gene locus. Briefly, a 2000 base pair sequence upstream of the AHL synthase gene and a 2000 base pair sequence downstream of the gene were amplified from genomic DNA with PCR. The fragments were appended to an antibiotic resistance cassette, containing its own promoter, terminator and ribosome binding site. The resulting linear construct was transformed into the *Methylobacillus flagellatus* strain by electroporation. A selection step on antibiotic containing, minimal medium agar plates with methanol as the carbon source was used to identify successful transformants. DNA integration into the correct locus was confirmed by colony PCR. The entire region after integration was amplified from the genome and checked by DNA sequencing. To ensure the AHL synthase gene was not present in the genome, a set of PCR primers was designed to target the gene specifically. Colony PCR utilizing these internal primers, that did not result in an amplified fragment was further evidence, that the AHL synthase gene was no longer present.

### Example 9 - Comparison of biomass production with or without AHL synthase deletion with carbon-limitation

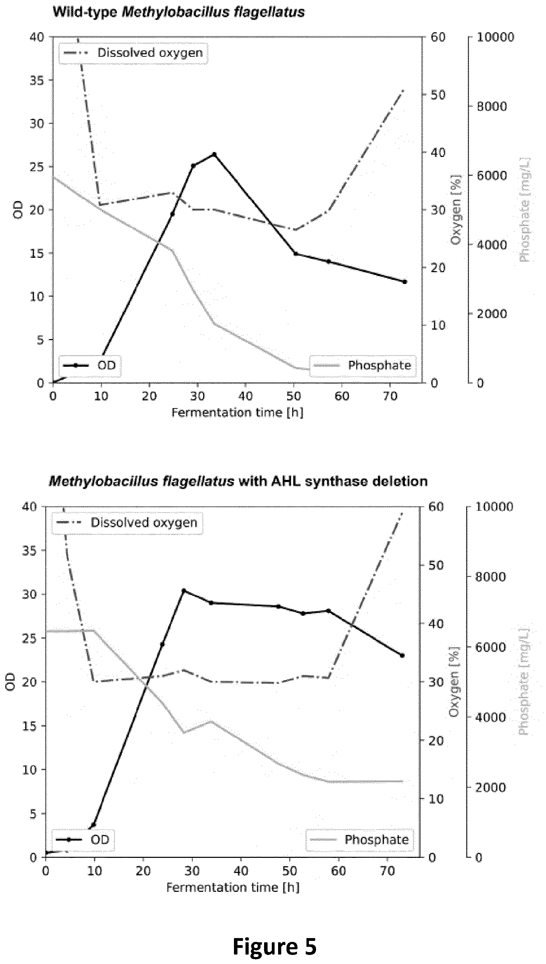

Side to side bioreactor fermentations were performed with *Methylobacillus flagellatus* strains with and without the AHL synthase gene (SEQ ID NO: 14). A mineral medium containing KH₂PO₄, Na₂HPO₄, MgSO₄, NH₄SO₄ and a trace element mixture was used. Carbon limitation was achieved by feeding methanol via a feedback loop connected to a dissolved oxygen sensor at a rate that maintained dissolved oxygen at 30% throughout the fermentation. The pH was kept at 7 by automatic addition of ammonium hydroxide, which also served as source of nitrogen. Bioprocess parameters such as optical density, CFU counts, whole-broth dry weight and free phosphate were measured regularly. In the Wild-type bioprocess, rapid cell lysis occurred once optical density reached ^{~}26, observed through a drop in optical density, a 99% decrease in colony forming units (CFU) and in the feeding rates of methanol and ammonium hydroxide. When the AHL synthase gene was not present, OD and CFU remained constant upon reaching stationary phase. Stationary phase could be maintained, during which product synthesis can occur **(****Figure 5****).**

### Example 10: Identification of EPS-producing genes in Methylobacillus flagellatus and Methylobacillus glycogenes

Several genomic loci were chosen for disruption based on bioinformatic analysis. The genetic locations of two EPS-associated gene clusters in *Methylobacillus flagellatus* have been published previously (Chistoserdova *et al*., 2007). To annotate genes in the first *Methylobacillus flagellatus* EPS-producing gene cluster (SEQ ID NOs: 113-138), homologues for genes from the published *Methylobacillus sp.* 12S methanolan synthesis cluster (Yoshida et al., 2000) were compared to the *Methylobacillus flagellatus genome* via an NCBI BLAST protein search, using a custom written script in the Python programming language. Top hits were selected based on a computed combination-score consisting of E-value, query coverage and sequence identity. A single top hit for each gene was then manually located in the genome sequence and annotated. Locations of these top hits coincided with the location of the published first cluster. Since a clear homologue was not found for each gene, all genes between the most distant homologues were considered to be part of the cluster. Most such non-annotated genes coded for sugar and polysaccharide related genes. The same procedure was used to identify the completely non-annotated EPS cluster in *Methylobacillus glycogenes* (SEQ ID NOs: 151-172).

A second EPS-related cluster was predicted in the genome sequence of *Methylobacillus flagellatus* (SEQ ID NOs: 139-150). A BLAST search comparing these genes to the methanolan cluster was performed, finding no clear homologues. The protein product of each individual gene in this cluster was identified via a NCBI protein BLAST search against all non-redundant protein sequences and the top hit for each was chosen as described above. The protein sequence of each gene from this *Methylobacillus flagellatus* protein cluster was cross-searched in the *Methylobacillus glycogenes* genome using NCBI protein BLAST. The top hits were found to form a similar cluster in *Methylobacillus glycogenes* (SEQ ID NOs: 173-198).

The identified open reading frames (ORFs) in each EPS-related gene cluster are shown in Tables 1 to 4 below.

**Table 1: ORFs of EPS gene cluster 1 in M. flagellatus**

| **Amino acid sequence SEQ ID NO** | **Nucleotide sequence SEQ ID NO** | **S12 homolog** | **Predicted function/ property** |
|---|---|---|---|
| 27 | 113 | *espA* | Transcriptional regulator, LuxR family |
| 28 | 114 | *espK* | Cyclic nucleotide-binding domain (cNMP-BD)protein |
| 29 | 115 | N/A | Unknown |
| 30 | 116 | *epsL* | Exosortase B-associated extracellular polysaccharide biosynthesis transporter EpsL |
| 31 | 117 | *epsB* | Transferase enzyme epsB family |
| 32 | 118 | *epsD* | Peptidyl-prolyl cis-trans isomerase, EpsD family |
| 33 | 119 | *epsE* | Polysaccharide export protein EpsE |
| 34 | 120 | *epsF* | Chain length determinant protein EpsF |
| 35 | 121 | *epsG* | Chain length determinant protein tyrosine kinase EpsG |
| 36 | 122 | *epsH* | Exosortase B |
| 37 | 123 | *epsI* | Epsl family protein |
| 38 | 124 | N/A | Polysaccharide biosynthesis protein |
| 39 | 125 | N/A | Polysaccharide pyruvyl transferase |
| 40 | 126 | N/A | Nitroreductase |
| 41 | 127 | N/A | O-antigen polymerase |
| 42 | 128 | *epsJ* | Glycosyl transferase, family 2 |
| 43 | 129 | N/A | Glycosyl transferase, family 2 |
| 44 | 130 | N/A | Glycosyl transferase, group 1 |
| 45 | 131 | N/A | Glycoside hydrolase, family 5 |
| 46 | 132 | N/A | Glycosyltransferase, RfaG |
| 47 | 133 | N/A | Mannose-1-phosphate guanylyltransferase (GDP) |
| 48 | 134 | N/A | dTDP-4-dehydrorhamnose reductase |
| 49 | 135 | N/A | dTDP-4-dehydrorhamnose 3,5-epimerase |
| 50 | 136 | N/A | Glucose-1-phosphate thymidylyltransferase |
| 51 | 137 | N/A | dTDP-glucose 4,6-dehydratase |
| 52 | 138 | *epsS* | UDP-galactose 4-epimerase |

**Table 2: ORFs of EPS gene cluster 2 in M. flagellatus**

| **Amino acid sequence SEQ ID NO** | **Nucleotide sequence SEQ ID NO** | **S12 homolog** | **Predicted function/ property** |
|---|---|---|---|
| 53 | 139 | N/A | Mannose-6-phosphate isomerase, type 2 |
| 54 | 140 | N/A | ABC transporter permease |
| 55 | 141 | N/A | NDP-hexose 3-C-methyltransferase TylCIII |
| 56 | 142 | N/A | GDP-mannose 4,6-dehydratase |
| 57 | 143 | N/A | NAD-dependent epimerase/dehydratase |
| 58 | 144 | N/A | Glycosyl transferase |
| 59 | 145 | N/A | Polysaccharide export protein |
| 60 | 146 | N/A | Glycosyl transferase |
| 61 | 147 | N/A | NAD-dependent epimerase/dehydratase |
| 62 | 148 | N/A | Sugar transferase |
| 63 | 149 | N/A | Polysaccharide biosynthesis protein CapD |
| 64 | 150 | N/A | UDP-glucose pyrophosphorylase |

**Table 3: ORFs of EPS gene cluster 2 in M. glycogenes**

| **Amino acid sequence SEQ ID NO** | **Nucleotide sequence SEQ ID NO** | **S12 homolog** | **Predicted function/ property** |
|---|---|---|---|
| 65 | 151 | *epsA* | Transcriptional regulator, LuxR family |
| 66 | 152 | *epsL* | Exosortase B-associated extracellular polysaccharide biosynthesis transporter EpsL |
| 67 | 153 | N/A | U ndeca prenyl-phosphate gl ucosephosphotra nsferase |
| 68 | 154 | *epsD* | Peptidyl-prolyl cis-trans isomerase, EpsD family |
| 69 | 155 | *epsE* | Polysaccharide export protein EpsE |
| 70 | 156 | *epsF* | Chain length determinant protein EpsF |
| 71 | 157 | *epsG* | Chain length determinant protein tyrosine kinase EpsG |
| 72 | 158 | *epsH* | Exosortase B |
| 73 | 159 | *epsI* | Epsl family protein |
| 74 | 160 | *epsB* | Glycosyltransferase |
| 75 | 161 | N/A | Oligosaccharide repeat unit polymerase |
| 76 | 162 | N/A | Right-handed parallel beta-helix repeat-containing protein |
| 77 | 163 | N/A | Acyltransferase family protein |
| 78 | 164 | N/A | Glycosyltransferase family 4 protein |
| 79 | 165 | N/A | Glycosyltransferase family 4 protein |
| 80 | 166 | N/A | Nucleotide sugar dehydrogenase |
| 81 | 167 | *epsR* | Unknown |
| 82 | 168 | N/A | Unknown |
| 83 | 169 | N/A | Oligosaccharide flippase family protein |
| 84 | 170 | N/A | Polysaccharide pyruvyl transferase family protein |
| 85 | 171 | N/A | Acyltransferase |
| 86 | 172 | *epsQ* | mannose-1-phosphate guanylyltransferase/mannose-6-phosphate isomerase |

**Table 4: ORFs of EPS gene cluster 2 in M. glycogenes**

| **Amino acid sequence SEQ ID NO** | **Nucleotide sequence SEQ ID NO** | **S12 homolog** | **Predicted function/ property** |
|---|---|---|---|
| 87 | 173 | N/A | MarR family EPS-associated transcriptional regulator |
| 88 | 174 | N/A | GDP-mannose 4,6-dehydratase |
| 89 | 175 | N/A | NAD-dependent epimerase/dehydratase family protein |
| 90 | 176 | N/A | Unknown |
| 91 | 177 | N/A | Polyhydroxyalkanoate synthesis repressor PhaR |
| 92 | 178 | N/A | UDP-N-acetylglucosamine 2-epimerase (hydrolyzing) neuC |
| 93 | 179 | N/A | Imidazole glycerol phosphate synthase subunit HisF |
| 94 | 180 | N/A | Imidazole glycerol phosphate synthase subunit HisH |
| 95 | 181 | N/A | N-acetyl sugar amidotransferase |
| 96 | 182 | N/A | Polysaccharide pyruvyl transferase family protein |
| 97 | 183 | N/A | Acyltransferase |
| 98 | 184 | N/A | Unknown |
| 99 | 185 | N/A | ABC transporter ATP-binding protein |
| 100 | 186 | N/A | FkbM family methyltransferase |
| 101 | 187 | N/A | Glycosyltransferase |
| 102 | 188 | N/A | Methyltransferase, TIGR04325 family |
| 103 | 189 | N/A | NAD-dependent epimerase/dehydratase family protein |
| 104 | 190 | N/A | DegT/DnrJ/EryC1/StrS family aminotransferase |
| 105 | 191 | N/A | Glycosyltransferase |
| 106 | 192 | *epsJ* | Glycosyltransferase |
| 107 | 193 | N/A | Glycosyltransferase |
| 108 | 194 | N/A | Glycosyltransferase |
| 109 | 195 | N/A | SDR family oxidoreductase |
| 110 | 196 | N/A | Sugar transferase |
| 111 | 197 | N/A | SDR family NAD(P)-dependent oxidoreductase |
| 112 | 198 | *epsT* | UTP--glucose-1-phosphate uridylyltransferase GalU |

**Table 5: Full sequences of clusters**

| **SEQ ID NO** | **Organism** | **Cluster** | **Strand** |
|---|---|---|---|
| 199 | *M. flagellatus* | 1 | top |
| 200 | *M. flagellatus* | 1 | bottom |
| 201 | *M. flagellatus* | 2 | top |
| 202 | *M. flagellatus* | 2 | bottom |
| 203 | M. glycogenes | 1 | top |
| 204 | M. glycogenes | 1 | bottom |
| 205 | M. glycogenes | 2 | top |
| 206 | M. glycogenes | 2 | bottom |

ORFs annotated as *epsD, epsE, epsF* and *epsG* (SEQ ID NO: 32-35 in *M*. *flagellatus*, SEQ ID NO: 68-71 in *M. glycogenes*) were chosen for deletion, based on their similarity to EPS-production essential genes in *Methylobacillus sp.* strain 12 (Yoshida *et al*., 2003). Since the second EPS cluster had no clear homologues in other characterized EPS clusters, all 12 ORFs in *M. flagellatus* (SEQ ID NO: 53-64) and all 26 ORFs in *Methylobacillus glycogenes* (SEQ ID NO: 87-112) were chosen for deletion.

### Example 11: DNA deletions

Modified strains of ABME 5 and ABME 6 were preparing in which either one EPS-producing cluster or both EPS-producing clusters are deleted. The genome edits were achieved by introducing a linear DNA fragment consisting a kanamycin or rifampicin resistance cassette flanked by 2 kb long sequences upstream and downstream of the integration sites. DNA fragments were transformed into ABME 5 and ABMR 6 by electroporation. Transformations were plated onto selective PM7 plates containing 20 g/L agar, 50 µg/mL kanamycin, or 5 µg/mL rifampicin for 24-48 h. ABME 5-derived strains were incubated at 30°C. ABME 6-derived strains were cultivated at 37°C. Correct clones were confirmed by colony PCR. The strains prepared by DNA deletion are listed in Table 6.

**Table 6: List of strains prepared with genomic integration.**

| **Strain** | **Species** | **Genotype** | **Deleted sequences** |
|---|---|---|---|
| ABME 5 | *Methylobacillus glycogenes* DSM 5685 | Wild type | |
| ABME 76 | *Methylobacillus glycogenes* DSM 5685 | epsDEFG :: kanR | SEQ ID NO: 207 |
| ABME 190TH | *Methylobacillus glycogenes* DSM 5685 | EPS2 :: kanR | SEQ ID NO: 208 |
| ABME 191TH | *Methylobacillus glycogenes* DSM 5685 | epsDEFG :: kanR, EPS2 :: rifR | SEQ ID NO: 207 and SEQ ID NO: 208 |
| ABME 6 | *Methylobacillus flagellatus* DSM 6875 | Wild type | |
| ABME 80 | *Methylobacillus flagellatus* DSM 6875 | EPS2 :: kanR | SEQ ID NO: 210 |
| ABME 82 | *Methylobacillus flagellatus* DSM 6875 | epsDEFG :: kanR | SEQ ID NO: 209 |
| ABME 131 | *Methylobacillus flagellatus* DSM 6875 | epsDEFG :: kanR, EPS2 :: rifR | SEQ ID NO: 209 and SEQ ID NO: 210 |
| ABME 145 | *Methylobacillus sp.* DSM 1758 | Wild type | |

### Example 12: Measurements of total EPS in shake flask cultures before and after EPS gene disruption in Methylobacillus flagellatus

To evaluate the effects of deleting EPS-producing genes in ABME 6, three modified ABME 6 strains (ABME 80, ABME 82 and ABME 131) were prepared as described in Example 11 and tested for EPS production.

Confirmed transformants were grown overnight on PM7 + 50 µg/mL kanamycin agar plates. Single colonies were resuspended in 25 mL PM7 medium + 25 µg/mL kanamycin in baffled 250 mL shake flasks, and incubated overnight at 200 rpm. Next day, 50 mL PM7 medium + 25 µg/mL kanamycin in baffled 250 mL shake flasks was inoculated with 2.5 mL overnight culture. All ABME 5-derived strains were cultivated at 30°C, and all ABME 6-derived strains were cultivated at 37°C. The cultures were then chilled on ice, aliquoted into 2 mL Eppendorf tubes and centrifuged for 15 minutes at 15 000 rpm on a standard tabletop centrifuge at 4 °C. When a layer of EPS was visible above the cell pellet, it was mixed back into the supernatant. The culture supernatants were stored for EPS hydrolysis. Hydrolysis was performed by adding half the volume of the supernatant of 6M TFA (trifluoroacetic acid), resulting in a final TFA concentration of 3M. Tubes were placed into a thermoblock heated to 120 °C and incubated for 6h. After hydrolysis, the liquid (including TFA) was evaporated at 80 °C until completely dry. The dry mass was resuspended in 0.5 M NaOH (at the same volume as the original culture aliquot) and analyzed for monomeric sugars on an HPLC.

**Table 7: Composition of PM7 medium used for shake-flask experiments**

| **Compound** | **Per 1L** |
|---|---|
| MeOH | 10 g |
| KH₂PO₄ | 0.65 g |
| NA₂HPO₄ | 1.05 g |
| MgSO₄ ^{∗} 7H₂O | 0.1 g |
| NH₄Cl | 0.2 g |
| (NH₄)₂SO₄ | 2g |
| TE1 1000X | 1 mL |
| d-Biotin | 0.1 mg |
| Thiamine^{∗}HCl | 0.1 mg |
| Riboflavin | 0.1 mg |
| Pyridoxine^{∗}HCl | 0.1 mg |
| Pantothenate | 0.1 mg |
| Nicotinamide | 0.1 mg |
| p-Aminobenzoic acid | 0.02 mg |
| Folic acid | 0.01 mg |
| Vitamin B12 | 0.01 mg |
| Lipoic acid | 0.01 mg |
| CaCl₂ ^{∗} 2H₂O | 3.3 mg |
| FeSO₄ ^{∗} 7H₂O | 1.3 mg |
| MnSO₄ ^{∗} H₂O | 0.1 mg |
| ZnSO4 ^{∗} 7H₂O | 0.13 mg |
| CuSO4 ^{∗} 5H2O | 0.04 mg |
| Na2MoO4 ^{∗} 2H2O | 0.04 mg |
| CoCl2 ^{∗} 6H2O | 0.04 mg |
| H3BO3 | 0.03 mg |
| pH | 7.0 |
| Agar (for solid media) | 20 g |

ABME 6 produced a visible slime layer on top of the cell pellet after centrifugation, amounting to ^{~} 1 g/L total monomeric sugars after hydrolysis. The single-cluster disruptions (ABME 80 and ABME 82) had no visible slime layer, but still produced ^{~} 500 mg/L total sugars. Interestingly, the glucose to total sugars ratio is inverted depending on which gene cluster was disrupted, most likely due to two different types of EPS being produced. When both clusters were disrupted (strain ABME 131), sugars were below the detection limit.

### Example 13: Measurements of viscosity in reactor cultures of Methylobacillus flagellatus before and after EPS gene disruption

To assess the change in fermentation broth viscosity before and after the disruption of EPS production, the strains were compared in 5-liter bioreactor fermentations under oxygen limitation. The bioreactor seed cultures were always prepared as follows. 25 mL of PM7 medium in 250 mL baffled shake-flasks were inoculated with 250 uL of strain cryo-stock with the stock OD adjusted to 5, to produce a starting OD of 0.05. The cultures were incubated overnight at 200 rpm 37°C. In the morning, 200 mL of PM7 medium in 2L baffled shake-flasks was inoculated with 10% (20 mL) of the overnight culture and grown until OD ^{~}1 (+/- 0.2). The culture was then used to inoculate a 2.5 L starting volume of sterile PM3 medium in 5-L bioreactors (8% inoculum). The pH was kept at 7 by automatic addition of NH₄OH and the temperature was kept at 37 °C.

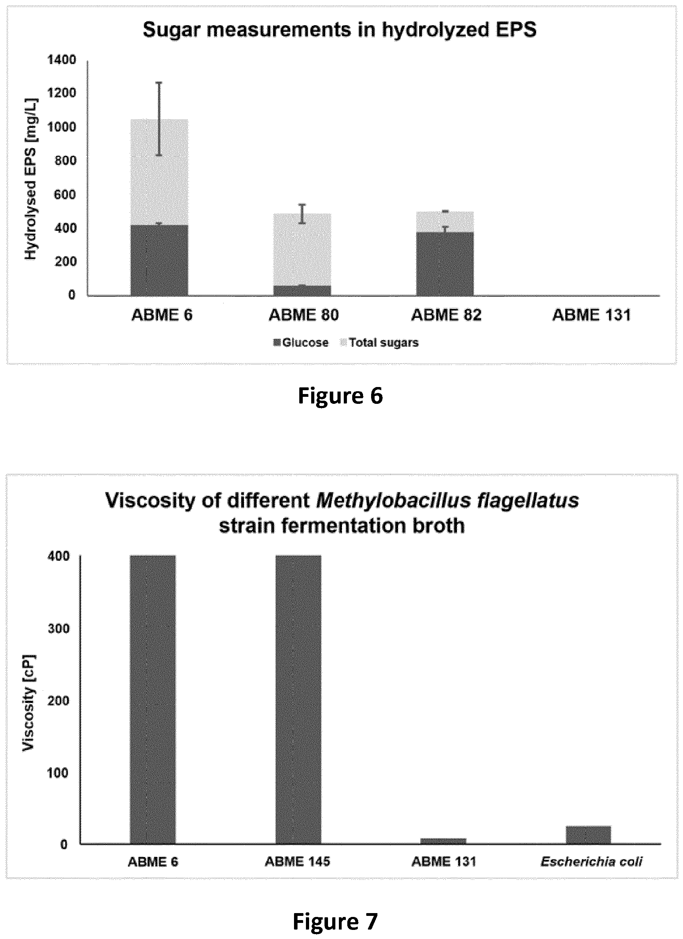

For oxygen limited processes, methanol was fed automatically to maintain a concentration between 3 and 6 g/L. Reactor aeration and stirring were kept at maximum, though the oxygen concentration always dropped to 0% during the process. The broth was sampled regularly for OD, mineral and metabolite measurements. Viscosity was measured at the end of the process and the results are summarized in **Figure 7**. Wild type *M. flagellatus* fermentation broth was highly viscous at the time of sampling at 400 centipoise. A different species of *Methylobacillus,* ABME 145 was measured as well and produced a similarly viscous broth of 422 centipoise. The double disruption produced a visibly less viscous broth, which could be centrifuged and filtered using standard tabletop equipment - something that was not possible with the previous samples. The viscosimeter confirmed this assessment, measuring in at 35 cP or a nearly 10-fold reduction in viscosity. A representative sample of *Escherichia coli* fermentation broth was also measured for comparison.

### Example 14: Measurements of reduced foaming in shake flasks before and after EPS gene elimination in Methylobacillus flagellates

We observed that the EPS-deficient strains showed significantly less foaming. To compare foam formation of ABME 6 against EPS-deficient mutant strains (ABME 82 and ABME 131), the strains were culture as described in Example 12.

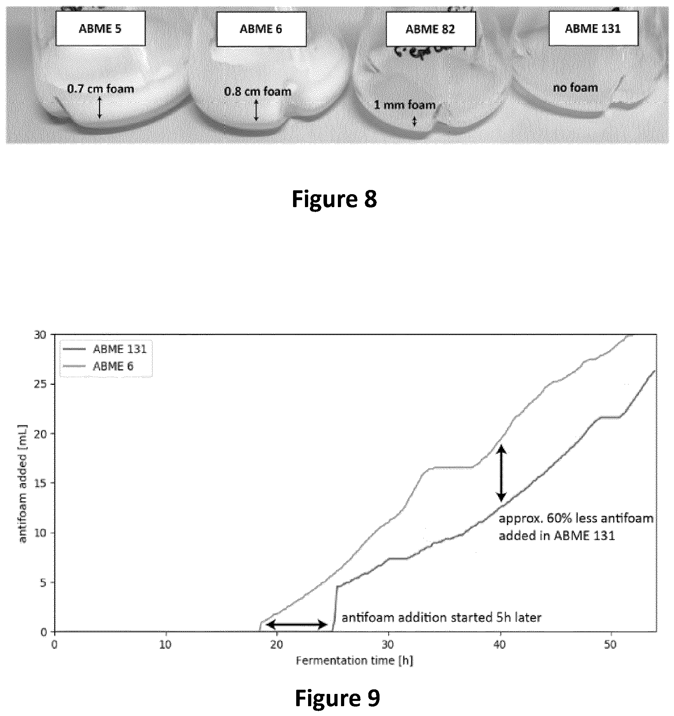

To measure foam formation flasks were taken off the shaker and the liquid was allowed to settle for 5 minutes. The foam layer on top of the liquid was measured with a tape measure (**Figure 8**). Wild type cultures produced 0.5 - 1 cm of foam layer on top of the liquid while the single-cluster disruptions only accumulated ^{~}1 mm of foam at the liquid meniscus. No foam at all was visible in the double-disruption cultures.

### Example 15: Measurements of reduced foaming in bioreactor cultivation before and after EPS gene disruption in Methylobacillus flagellatus

To assess the change in fermentation broth foaming before and after the disruption of EPS production, the strains were compared in 5-liter bioreactor fermentations. The amount of foam formation was approximated by the amount of added anti-foaming agent (SAG 5693). The fermentations were carried out under carbon limitation.

The reactors were inoculated as described in Example 12. To achieve carbon limitation, 50% methanol was fed to the reactor automatically at a dynamically-calculated rate that that maintained pO2-value at approximately 30% at the highest aeration and stirring settings. The added methanol was consumed immediately and kept below detection levels. Antifoam was added automatically when foam reached a detector placed above the fermentation broth meniscus. ABME 6 and ABME 131 were tested in this way. Antifoam addition started 5h later in the case of ABME 131. Approximately 60% less antifoam was added in the ABME 131 fermentation, depending on the fermentation time (**Figure 9**).

### Example 16: Improved methanol tolerance of EPS-free M. flagellates

Tolerance to high methanol concentrations is a critical parameter for efficient bioprocesses. To confirm that methanol tolerance was not reduced in strains that cannot produced EPS, growth at increasing methanol concentrations was tested in ABME 6 and its EPS-producing derivative strain ABME 131.

The strains were tested in shake flasks as described in Example 13. To test methanol tolerance, the initial methanol concentration was increased up to 4.5 %. OD600 was measured after 24h.

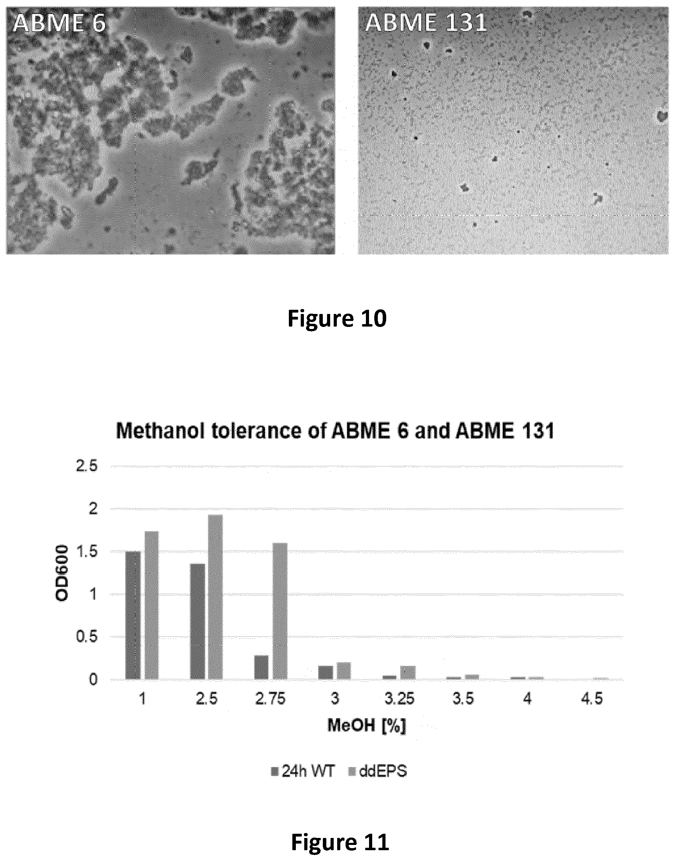

Surprisingly, the methanol tolerance of ABME 131 was higher compared to ABME 6. For example, at 2.75 % initial methanol concentration, the EPS-free ABME 131 grew to an OD600 of 1.6, whereas the wild-type ABME 6 only grew to an OD600 of 0.3. The difference in growth was most striking at 2.75 %. However improved growth of ABME 131 compared to ABME 6 was apparent at all methanol concentration (**Figure 11**).

### List of references cited in the description

Pennacchietti E, Lammens TM, Capitani G, Franssen MC, John RA, Bossa F, De Biase D., 2009. Mutation of His465 alters the pH-dependent spectroscopic properties of Escherichia coli glutamate decarboxylase and broadens the range of its activity toward more alkaline pH. J Biol Chem. 284(46):31587-96. doi: 10.1074/jbc.M109.049577.

Thu Ho, N.A., Hou, C.Y., Kim, W.H., Kang, T.J., 2013. Expanding the active pH range of Escherichia coli glutamate decarboxylase by breaking the cooperativeness. J. Biosci. Bioeng. https://doi.org/10.1016/j.jbiosc.2012.09.002

Yu, K., Lin, L., Hu, S., Huang, J., Mei, L., 2012. C-terminal truncation of glutamate decarboxylase from Lactobacillus brevis CGMCC 1306 extends its activity toward near-neutral pH. Enzyme Microb. Technol. 50, 263-269. https://doi.org/10.1016/j.enzmictec.2012.01.010

Blin, K. et al. (2021) "antiSMASH 6.0: improving cluster detection and comparison capabilities", Nucleic Acids Res., 49(W1): W29-W35. doi: 10.1093/nar/gkab335.

Chistoserdova, L. et al. (2007) 'Genome of Methylobacillus flagellatus, Molecular Basis for Obligate Methylotrophy, and Polyphyletic Origin of Methylotrophy', Journal of Bacteriology. American Society for Microbiology Journals, 189(11): 4020-4027.

Yoshida, T. et al. (2000) 'Saccharide production from methanol by transposon 5 mutants derived from the extracellular polysaccharide-producing bacterium Methylobacillus sp. strain 12S', Applied Microbiology and Biotechnology. doi: 10.1007/s002530000407.

Yoshida, T. et al. (2003) 'Genes involved in the synthesis of the exopolysaccharide methanolan by the obligate methylotroph Methylobacillus sp. strain 12S', Microbiology. Microbiology Society, pp. 431-444. doi: 10.1099/mic.0.25913-0.

Qiu Z and Goodman MF: The Escherichia coli polB locus is identical to dinA, the structural gene for DNA polymerase II. Characterization of Pol II purified from a polB mutant. J Biol Chem. 1997, 272(13): 8611-8617.

Kwon DH, Peña JA, Osato MS, Fox JG, Graham DY, Versalovic J: Frameshift mutations in rdxA and metronidazole resistance in North American Helicobacter pylori isolates. J Antimicrob Chemother 2000, 46(5): 793-796

Datsenko KA, Wanner BL: One-step inactivation of chromosomal genes in Escherichia coli K-12 using PCR products. Proc Natl Acad Sci U S A 2000, 97:6640-6645.

Zhu Y, Huang W, Lee SS, Xu W. Crystal structure of a polyphosphate kinase and its implications for polyphosphate synthesis. EMBO Rep. 2005; 6(7):681-7.

Tzeng CM, Kornberg A. The multiple activities of polyphosphate kinase of Escherichia coli and their subunit structure determined by radiation target analysis. J Biol Chem. 2000; 275(6):3977-83.

## Claims

1. A genetically engineered bacterium which has been modified to have a decreased expression and/or activity of an endogenous polypeptide having polyphosphate kinase activity compared to an otherwise identical bacterium that does not carry said modification.

2. The bacterium according to claim 1, which has been modified to have a decreased expression of an endogenous polypeptide having polyphosphate kinase activity compared to an otherwise identical bacterium that does not carry said modification.

3. The bacterium according to claim 1 or 2, wherein the endogenous gene encoding said polypeptide having polyphosphate kinase activity has been inactivated.

4. The bacterium according to any one of claims 1 to 3, which has been further modified to have a decreased expression and/or activity of an endogenous polypeptide having Acyl-homoserine-lactone (AHL) synthase activity compared to an otherwise identical bacterium that does not carry said modification.

5. The bacterium according to any one of claims 1 to 4, which has been modified to have a decreased expression of an endogenous polypeptide having Acyl-homoserine-lactone (AHL) synthase activity compared to an otherwise identical bacterium that does not carry said modification.

6. The bacterium according to claim 4 or 5, wherein the endogenous gene encoding said polypeptide having Acyl-homoserine-lactone (AHL) synthase activity has been inactivated.

7. The bacterium according to any one of claims 1 to 6, which is a mesophilic, methylotrophic bacterium.

8. The bacterium according to any one of claims 1 to 7, wherein said bacterium belongs to the genus *Methylobacillus, Methylobacterium or Methylorubrum.*

9. The bacterium according to any one of claims 1 to 8, which is of the genus *Methylobacillus.*

10. The bacterium according to any one of claims 1 to 9, which is selected from *Methylobacillus flagellatus*, *Methylobacillus glycogenes, Methylobacillus pratensis, Methylobacillus rhizosphaerae, Methylobacillus gramineus*, *Methylobacillus arboreus*, *Methylobacillus caricics, Methylobacillus methilovorans and Methylobacillus sp.*

11. The bacterium according to any one of claims 1 to 10, wherein said bacterium is *Methylobacillus flagellatus or Methylobacillus glycogenes.*

12. The bacterium according to any one of claims 9 to 11, which has been modified to have a decreased production of exopolysaccharides (EPS) compared to an otherwise identical bacterium that does not carry said modification.

13. The bacterium according to claim 12, which has been modified to have a decreased expression and/or activity of at least one endogenous polypeptide involved in the production of exopolysaccharides (EPS) in said bacterium compared to an otherwise identical bacterium that does not carry said modification.

14. Method for producing a biochemical comprising cultivating a bacterium according to any one of claims 1 to 13 under suitable culture conditions.

15. Method for producing biomass comprising cultivating a bacterium according to any one of claims 1 to 13 under suitable culture conditions.
